# EUROPEAN PATENT APPLICATION

(11) **EP 2 574 332 A1**
(43) Date of publication of application: **03.04.2013**
(21) Application number: 12189355.6
(22) Date of filing: 06.06.2008
(51) Int. Cl.: A61K 8/97, A61Q 19/08, A61P 17/00, A61K 31/437, A61K 36/28

(54) **MC-1 R, MC-2 R, and µ opioid receptors modulation**

(30) Priority: 06.06.2007 FR 0755529
(62) Divisional of application: 08760699.2
(71) Applicant: BASF Beauty Care Solutions France SAS, 69007 Lyon (FR)
(72) Inventor: Pain, Sabine, 38200 VIENNE (FR); Dezutter, Colette, 38200 VIENNE (FR); Andre, Valérie, 69420 AMPUIS (FR); Reymermier, Corinne, 69390 CHARLY (FR); Orly, Isabelle, 69540 IRIGNY (FR); Perrier, Eric, 38138 LES COTES D'AREY (FR)
(74) Representative: Dorff, Gernot Peter Roderich

(57) **Abstract**

The invention concerns active substances modulating the expression of the receptors of the products of the POMC gene (MC-1R, MC-2R, and the µ opioid receptor), and possibly modulating the expression of POMC in cutaneous cells, notably to modulate the proliferation and differentiation of epidermal cells in order to re-epithelial, maintain innervation, irrigate the skin, or restore normal pigmentation, or to fight against aging, or to modulate pigmentation independently or not of cutaneous aging.

The invention also concerns a method of screening such active substances.

## Description

The invention concerns the active components modulating the expression of neuromediating receptors coded by the POMC (proopiomelanocortin) gene and possibly modulating the expression of corresponding neuromediators. The invention also addresses the use of a composition containing at least one substance active in the expression of neuromediating receptors coded by the POMC gene at the cutaneous level, as well as a method of screening such active components.

### Current understanding of the subject:

Due to its sensory functions, the skin is a primordial source of information for the individual. Peripheral nerves are responsible for the innervation of the skin and they possess axons whose cellular bodies are located along the spinal cord. Cutaneous innervation includes, among others, sensory nerves and autonomous sympathetic fibers. Superficial free nerves endings are the only sensory fibers that penetrate to the interior of the epidermis. An important exchange exists between skin cells and cutaneous nerves. Skin cells synthesize numerous neuromediators that act on the skin neurons (paracrine route). Cutaneous cells also possess receptors corresponding to certain neuromediators released by nerve cells and the cutaneous cells themselves; they can thus receive and respond to these neuromediators.

Skin aging is associated with a deregulation of the metabolism of cutaneous cells characterized by a diminishing of the proliferation of keratinocytes, a deregulation of the differentiation of keratinocytes, an accumulation of dead cells, and a diminishing of the innervation of the skin.

Alpha-MSH or alpha-melanocyte-stimulating-hormone (α-MSH), adrenocorticotropic hormone (ACTH), beta lipotropic (β-LPH), and beta endorphins are neuro-hormones generated by the single gene proopiomelanocortin (POMC). They are mainly synthesized in the pituitary gland but are also found in numerous peripheral tissues such as the skin (Wintzen M, Yaar M, Burbach JP, Gilchrest BA. J Invest Dermatol. 1996 Apr; 106(4):673-8*.).* The POMC gene and the proteins α-MSH, ACTH, β-LPH and beta endorphin are expressed by dermal cells, fibroblasts, Langerhans cells, and epidermal cells, melanocytes, and keratinocytes.

To these proteins correspond receptors which are also expressed by skin cells. Melanocortin receptor 1 (MC-1R or MCR-1) is the receptor of α-MSH and ACTH, melanocortin receptor 2 (MC-2R or MCR-2) is the receptor of ACTH, and µ opioid receptor (µ opioid R) is the receptor of β endorphin.

In the skin, α-MSH, ACTH and β endorphin inhibit the synthesis of the inflammatory cytokines IL-1, TNFα, and IL-10; they thus have anti-inflammatory properties. Moreover, these same cytokines stimulate the synthesis of these neuro-hormones *(*Moustafa M, Szabo M, Ghanem GE, Morandini R, Kemp EH, MacNeil S, Haycock JW. J Invest Dermatol. 2002 Dec; 119(6):1244-53*.).* Thus, there exists in the skin a negative retro-control of neurocutaneous inflammation.

α-MSH and ACTH are known to regulate melanogenesis by acting on their respective receptors in melanocytes. α-MSH and ACTH are inductors of melanocyte proliferation. In addition, β endorphin is known to have a mitogenic effect on melanocytes and increases melanocyte dendricity *(*Kauser S, Schallreuter KU, Thody AJ, Gummer C, Tobin DJ, J Invest Dermatol. 2003 Jun; 120(6):1073-80*) .*

α-MSH and β endorphin are known to be synthesized by keratinocytes as well. These two proteins induce the proliferation and differentiation of skin keratinocytes *(*Chakraborty AK, Funasaka Y, Slominski A, Ermak G, Hwang J, Pawelek JM, Ichihashi M., Biochim Biophys Acta. 1996 Aug 28; 1313(2):130-8*).*

The different cells of the skin (keratinocytes, melanocytes, and fibroblasts) are capable of synthesizing other neuromediators such as neurotrophins like nerve growth factor (NGF). Keratinocytes and fibroblasts produce receptors to NGF, TrkA, and p75NTR. By attaching itself to TrkA, NGF induces the proliferation of these cells and protects keratinocytes from apoptosis. NGF is widely known for inducing neuron differentiation and permitting their survival; thus it plays an important role in the maintenance of neuronal density. Moreover, in mice the presence of the POMC mRNA has been shown in the neurons of the ganglions of the dorsal root, which suggests the involvement of the melanocortin system and notably of α-MSH in the repair of peripheral nerves. Alpha MSH would thus play a role in neurotrophicity *(*Gispen WH, Adan RA. Ann N Y Acad Sci. 1999 Oct 20;885:342-9*,* van der Kraan M, Tatro JB, Entwistle ML, Brakkee JH, Burbach JP, Adan RA, Gispen WH. Brain Res Mol Brain Res. 1999 Jan 8;63(2):276-86*).* Moreover, in attaching itself to the µ opioid receptors of nervous cells, beta endorphin and its derivatives are known to produce a sensation of well-being.

Up to now, professionals have sought to modulate the release of neuromediators such as α MSH or β endorphins or to mimic their role in order to affect homeostasis of the skin and/or improve innervation of the skin.

In addition, professionals are usually seeking to combat the effects of cutaneous aging through different metabolic routes that are not all comparable. However, today there remain new paths to explore.

A previous patent application WO2007/039058 has thus disclosed general use of opioid receptor antagonists to down-regulate the expression of the opioid receptors in melanocytes. Another previous patent application US2004/0214851 has disclosed a method for increasing expression of opioid receptors for diagnostic and/or therapeutic utility thus only in damaged tissues. Thus these disclosures do not provide with solutions to fight the effects of cutaneous aging as previously defined or recited herein after.

### Goals of the invention:

The inventors have sought innovative ways to restore innervation of the skin or maintain or promote homeostasis of skin cells, notably as part of the general context of aging and of its pigmentary flaws.

The invention is also intended to provide criteria permitting the evaluation of the impact of active components in dermato-cosmetology on the innervation of the skin, homeostasis of the skin, and melanogenesis.

The main goal of the invention is to provide at least one active substance, notably at the cutaneous level, in order to:
- Promote cellular homeostasis, in particular encouraging the proliferation of keratinocytes, in order to permit re-epithelialization,
- Directly or indirectly restore or maintain innervation of the skin in order to permit maintenance of the neuritic network of the epidermis and to procure a feeling of well-being,
- Directly or indirectly restore or maintain angiogenesis in order to maintain satisfying blood irrigation,
- Regulate melanogenesis.
All of this is in order to prevent orand/ fight against skin aging and/or the effects of stress inducing variations observed during cutaneous aging, for example when aging is photo-induced or chronological.

Thus one of the goal of this invention is to prevent and/or fight against the loss of properties of the skin, particularly of the epidermis, and to prevent and/or fight against the changing of the natural coloration of the skin, notably in the form of pigmentary spots, for example white or brown.

This invention is also intended to provide a substance permitting the cascade induction of the synthesis of other mediators involved in the innervation, vascularization, and epithelialization of the skin.

This invention is also intended to provide a substance to prevent and/or fight against a loss of trophicity at the cutaneous level.

This invention is also intended to provide active substances such as described above in the domain of cosmetics, and/or pharmacy, notably dermatology.

A notable goal of this invention is to provide active substances applicable topically and/or neutraceutically, in particular at least one plant extract, possibly transformed via biotechnology, or at least one characterized molecule.

By biotechnology, the inventors mean a process comprising at least one step carried out in the presence of a microorganism.

### Summary of the invention:

Among the innovative paths explored by the inventors to palliate the technical problems mentioned above and attain the goals of this invention, the inventors have discovered that it was particularly interesting to modulate and more preferably to increase, maintain or decrease the expression of at least one receptor of at least one mediator coded by the POMC gene at the cutaneous level.

Methods of identifying active components are based on the induction of the expression of the hormones α MSH or β endorphin or on the addition of substances that imitate the action of these neurohormones but do not in any way cause the expression of the receptors of these hormones.

The inventors have discovered that there was approximately a around 4-times diminishing of expression at the level of the genes of the receptors MC-1R, MC-2R, and µ opioid R and at around 5-times increase of the POMC gene in keratinocytes during the process of chronobiological aging. In addition, since MC-1R and µ opioid receptors are expressed by melanocytes, the active substances of this invention are used to prevent and/or fight against cutaneous aging and/or modify skin pigmentation.

This invention concerns the substances modulating the expression of at least one of the 3 receptors MC-1R, MC-2R, and µ opioid R, particularly at the cutaneous level, in order to improve the effect of the neuromediator that will thus permit the cascade induction of the synthesis of other mediators involved in the innervation, vascularization, and epithelialization of the skin.

The invention concerns at least one substance modulating the expression of at least one of the genes of the 3 receptors MC-1R, MC-2R, and µ opioid R, particularly at the cutaneous level, in order to maintain or promote cellular homeostasis at the cutaneous level, to restore innervation or blood irrigation of the skin, and to regulate melanogenesis.

By modulation of the expression of at least one receptor MC-1R, MC-2R, or µ opioid receptor, the inventors mean the modulation (stimulation or inhibition, possibly partial) of at least one gene coding the protein MC-1R, MC-2R, or µ opioid R, respectively, but also the modulation (stimulation or inhibition, possibly partial) of the synthesis of at least the protein MC-1R, MC-2R, or the µ opioid receptor from the respective RNA messengers, as well as the modulation (stimulation or inhibition, possibly partial) of the activity of the proteins MC-1R, MC-2R, or µ opioid R.

By stimulation of the expression of at least one receptor MC-1R, MC-2R, or µ opioid receptor, the inventors mean the stimulation, possibly partial of at least one gene coding the protein MC-1R, MC-2R, or µ opioid R, respectively, but also the stimulation, possibly partial, of the synthesis of at least the protein MC-1R, MCR2, or the µ opioid receptor from the respective RNA messengers, as well as the stimulation, possibly partial, of the activity of the proteins MC-1R, MC-2R, or µ opioid R.

By inhibition of the expression of at least one receptor MC-1R, MC-2R, or µ opioid receptor, the inventors mean the inhibition, possibly partial of at least one gene coding the protein MC-1R, MC-2R, or µ opioid R, respectively, but also the inhibition, possibly partial, of the synthesis of at least the protein MC-1R, MCR2, or the µ opioid receptor from the respective RNA messengers, as well as the inhibition, possibly partial, of the activity of the proteins MC-1R, MC-2R, or µ opioid R

By the term "restore" the inventors mean, in reference to a parameter, the fact of returning from a level of this parameter inferior or superior to what would be desirable for good physiological functioning to a physiological level that is more favorable to the subject concerned.

By the term "procure a feeling of well-being" the inventors mean the well-being provoked by the liberation of beta endorphins that attach themselves easily to the µ-opioid receptor.

This invention particularly concerns the modulation and more preferably the stimulation of the expression of the MC-1R gene and/or the MC-2R gene and/or the µ opioid R gene, and/or the proteins respectively coded by these genes, particularly in human beings.

This invention also particularly concerns the modulation of products of the POMC gene as well as the MC-1R and/or MC-2R receptors, and/or µ opioid R, in order to increase the effectiveness of receptor-ligand complexes; thus the active components also modulating the expression of the POMC gene are still preferable. It is preferable to stimulate or maintain or decrease the expression of the POMC gene in the case of melanogenesis to modify pigmentation.

The modulation should preferably be sufficiently effective to permit the stimulation of the proliferation and/or differentiation of the targeted cutaneous cells that express at least one of these receptors, and/or restore innervation of the skin at least partially.

Active components permitting the obtaining of an expression equal to at least around 1.2 times and preferably at least 2 times the expression of the genes coding MC-1R, MC-2R, and/or the µ opioid receptor in a model comprising at least one cellular type presenting the expression of these receptors in contact with these active components in comparison with the level of expression of these receptors in a control model (one not put in contact with the active components) are considered as effectively stimulating or activating the genes of the receptors mentioned above.

Active components permitting the obtaining of an expression equal to at least approximately 1.1 times, and advantageously 1.2 times the expression of the proteins MC-1R, MC-2R, and/or the µ opioid R in a model comprising at least one cellular type presenting the expression of these receptors in contact with these active components in comparison with the level of expression of these receptors in a control model (one not put in contact with the active components) are considered as effectively stimulating or activating the expression of the receptors mentioned above.

The active components permitting the obtaining of an expression lesser than or equal to around 0.95 and advantageously 0.8 times the expression of the genes MC-1R, MC-2R, and/or the µ opioid R in a model comprising at least one cellular type presenting the expression of these receptors in contact with these active components in comparison with the level of expression of these receptors in a control model (one not put in contact with the active components) are considered as effectively inhibiting or diminishing the expression of the receptors mentioned above.

The active components permitting the obtaining of an expression lesser than or equal to around 0.95 and advantageously 0.8 times the expression of the genes MC-1R, MC-2R, and/or the µ opioid R in a model comprising at least one cellular type presenting the expression of these receptors in contact with these active components in comparison with the level of expression of these receptors in a control model (one not put in contact with the active components) are considered as effectively inhibiting or diminishing the expression of the receptors mentioned above.

According to a specific method of execution it is preferable to activate or stimulate the expression of the genes MC-1R, MC-2R, and/or the µ opioid receptor, and/or the proteins MC-1R, MC-2R and/or the µ opioid receptor, and possibly maintain or stimulate the POMC gene and/or the neuromediators coded by the POMC gene in the keratinocytes and /or in nervous cells notably in cutaneous nervous network.

According to another method of execution, the expression of at least one of these expressed receptors is inhibited or diminished in keratinocytes in order to diminish the proliferation of keratinocytes, notably as part of a pathology related to a hyperproliferation of these cutaneous cells. Preferably the expression of at least one of the expressed receptors MC-1R, MC-2R and/or the µ opioid receptor is stimulated in keratinocytes in order to decrease inflammatory response notably associated with pathology of these cutaneous cells including pathologies related to a hyperproliferation of these cutaneous cells.

According to another specific method of execution, the expression of the genes MC-1R and/or the µ opioid receptor, and/or the proteins MC-1R and/or the µ opioid receptor, and possibly the expression of the POMC gene and/or the neuromediators coded by the POMC gene, is modulated in melanocytes. According to one embodiement, the expression of the genes MC-1R and/or the µ opioid receptor, and/or the proteins MC-1R and/or the µ opioid receptor are decreased for whitening effect, and possibly the expression of the POMC gene and/or the neuromediators coded by the POMC gene is additionally maintained or decreased. According to another embodiement, the expression of the genes MC-1R and/or the µ opioid receptor, and/or the proteins MC-1R and/or the µ opioid receptor are stimulated for pigmenting effect, and possibly the expression of the POMC gene and/or the neuromediators coded by the POMC gene is additionally maintained or stimulated

One aspect of this invention concerns a method of identification of an active substance modulating the proliferation and differentiation of at least one type of living cells capable of expressing MC-1R, MC-2R, and/or characterized µ opioid receptor characterized in that it includes:
- The putting in contact of the active substance with at least one type of living cells capable of expressing MC-1R, MC-2R, and/or the µ opioid receptor, and possibly the POMC gene;
- Analysis of the expression of MC-1R, MC-2R, and/or µ opioid receptor, with the particular objective of identifying an active substance modulating, preferably stimulating the expression of MC-1R, MC-2R and/or µ receptor.

Advantageously, the analysis of the expression of the genes of the receptors MC-1R, MC-2R, and/or the µ opioid receptor, and possibly of the POMC gene, is carried out by qualitative and/or quantitative analysis of the expression of a sequence of nucleotides coding MC-1R, MC-2R, and/or the µ opioid receptor.

Advantageously, the RT-PCR includes the use of primers hybridizing with at least one part of the sequence of DNA nucleotides complementary to the SEQ ID No. 1, 2, 3, 4 to amplify at least one part of the sequence of nucleotides coding MC-1R, MC-2R, the µ opioid receptor, and possibly the POMC gene.
SEQ ID N°1: MC-1R NM_002386
SEQ ID N° 2: MC-2R NM_000529
SEQ ID N° 3: µ opioid receptor NM_000914
SEQ ID N° 4: POMC NM_000939

Preferably, the living cells used include keratinocytes and/or melanocytes and/or nervous cells including cutaneous nervous network notably extracted from the skin of an adult human subject, this skin having a specific location (abdomen, breast, etc). It is preferable to use so-called "normal" cells; that is, for example, non-transformed cells (tumorous or genetically modified) and significantly representative for tests of the cellular type of the group of subjects being studied.

Preferably, categories of age are created in order to establish an age/genetic expression relationship and age/protein synthesis relationship. The subjects referred to as young are between 17 and 40 years of age; the subjects referred to as intermediate are between 40 and 50 years of age, and the older subjects are older than 50 years.

Preferably, the method of identification comprises a step of analysis of the expression of the proteins MC-1R (34.7 kDa), MC-2R (33.9 kDa), and/or µ opioid receptor (44.8 kDa) via the method of transfer of proteins from a gel to a nitrocellulose membrane (Western blot) notably in order to detect modulation of the expression of the corresponding proteins when the active substance is in contact with the aforementioned living cells.

According to first preferred method of execution, the type of living cells capable of expressing MC-1R, MC-2R, and/or µ opioid receptor are keratinocytes. In this case, the active components are sought that stimulate or increase the expression of MC-1R, MC-2R, and/or µ opioid receptor, and possibly diminishing the expression of the POMC gene.

According to a second method of execution, the type of living cells capable of expressing MC-1R and/or µ opioid receptor are melanocytes. In this case, the active components are sought that modulate the expression of MC-1R and/or µ opioid receptor, and possibly modulating the expression of the POMC gene.

Advantageously, in acting on keratinocytes and/or nerve cells, including cutaneous nervous network, the active substance induces a cascade increase of NGF and VEGF synthesises, and thus stimulates innervation (density and dendricity of neurons) and neoangiogenesis, respectively.

A second aspect of the invention concerns the use of an active substance as described previously; that is, which can be identified as being active according to the screening method described above, as an active substance in a cosmetic, or neutraceutic composition, or for the manufacture of a pharmaceutical preferably dermatological composition, notably in order to modulate, preferably stimulate or decrease the synthesis of the proteins MC-1R and/or MC-2R and/or µ opioid receptor, notably in order to induce the proliferation and/or differentiation and/or maturation of keratinocytes and/or of melanocytes and/or of nerve cells, notably in cutaneous nervous network.

A third invention of the aspect concerns the use of an active substance as previously described as active substance in a cosmetic, or neutraceutic composition, or for the manufacture of a pharmaceutical preferably dermatological composition, notably to prevent and/or fight against chronobiological cutaneous aging or that caused by solar rays and/or against the effects of stress inducing the variations observed during the process of cutaneous aging, or prevent and/or fight against the diminishing of epidermal homeostasis, or to encourage epithelialization, or to improve cell proliferation and differentiation, notably at the epidermal level, or to prevent or fight against a diminishing of cutaneous vascularization, or to improve skin vascularization, or to improve vascular hyperpermeability, or to improve angiogenesis during the scarring of the skin, or to prevent and/or fight against the diminishing of skin innervation, or to improve skin innervation, or to generate a sensation of well-being, or to improve the color and/or tint of the skin and/or modify skin pigmentation, notably by increasing or decreasing skin pigmentation, in particular when this pigmentation presents localized flaws such as pigmentary spots.

A fourth aspect of the invention concerns a cosmetic or neutraceutic composition to prevent and/or fight against cutaneous aging and/or against the effects of stress inducing the variations observed during cutaneous aging, or prevent and/or fight against the diminishing of epidermal homeostasis, or to encourage epithelialization, or to improve cellular proliferation and differentiation, notably at the epidermal level, or to prevent or fight against a diminishing of cutaneous vascularization, or to improve skin vascularization, or to improve vascular hyperpermeability, or to improve angiogenesis during the scarring of the skin, or to prevent and/or fight against a diminishing of the innervation of the skin, or to improve skin innervation, or to generate a sensation of well-being, or to improve the color and/or tint of the skin and/or modify skin pigmentation, notably by increasing or decreasing skin pigmentation, in particular when the pigmentation presents localized flaws such as pigmentary spots; the aforementioned composition including as an active ingredient an active substance as previously defined, possibly in combination with a substance as previously described.

A fifth aspect of the invention concerns a pharmaceutical composition, preferably intended to prevent and/or fight against cutaneous aging and/or against the effects of stress inducing variations observed during the course of cutaneous aging, or prevent and/or fight against the diminishing of epidermal homeostasis, or to encourage epithelialization, or to improve cellular proliferation and differentiation, notably at the epidermal level, or to prevent and/or fight against a diminishing of cutaneous vascularization, or to improve vascular hyperpermeability, or to improve angiogenesis during the scarring of the skin, or to prevent or fight against the diminishing of skin innervation, or to improve skin innervation, or to generate a sensation of well-being, or to improve skin color and/or tint and/or modify skin pigmentation, notably by increasing or decreasing skin pigmentation, in particular when that pigmentation presents localized flaws such as pigmentary spots;the aforementioned composition including as an active ingredient an active substance such as previously defined, possibly in combination with a substance such as previously described.

A sixth aspect of the invention concerns a method of cosmetic care characterized in that a cosmetic composition as previously defined, or an active substance as previously defined is applied in the skin fro improved esthetic effects.

The effective quantity of active substance referred to is determined by the professional through simple routine experience.

The invention also concerns a method of prophylactic or therapeutic treatment as part of the whole of the goals and applications noted above.

### Detailed description of the invention:

The inventors have shown for the first time that the expression of the receptors MC-1R, MC-2R, and µ opioid R diminishes during the process of chronobiological cutaneous aging, notably when these receptors are expressed by keratinocytes in human skin.

The inventors have also identified for the first time that the expression of the POMC gene is increased during chronobiological cutaneous aging, notably in the keratinocytes in human skin.

The inventors have thus developed a screening process on keratinocytes to permit the search for actives, notably among vegetable extracts or characterized chemical molecules or molecules transformed by biotechnology, stimulating in particular the expression of mRNAs coding the receptors MC-1R, MC-2R, and µ opioid R, and possibly maintaining, stimulating or inhibiting the expression of mRNAs coding the neuromediators coded by the POMC gene. The actives selected were then tested on the expression of the proteins corresponding to the mRNAs.

The inventors have also characterized the expression of the MC-1R, MC-2R, and µ opioid receptors in keratinocytes. Only the mRNA of MC-2R has previously been identified in keratinocytes *(*Moustafa M, Szabo M, Ghanem GE, Morandini R, Kemp EH, MacNeil S, Haycock JW., J Invest Dermatol. 2002 Dec; 119(6):1244-53*.),* thus the inventors are the first to have identified the protein MC-2R in keratinocytes, implying the fact that ACTH carries out its activity not only by attaching itself to MC-1R in keratinocytes, but also in attaching itself to MC-2R.

Thus this invention describes the use of a substance stimulating the expression of at least one gene coding at least one receptor of a neuromediator coded by the POMC gene preferably chosen from among MC-1R, MC-2R and µ opioid R in at least one type of skin cells expressing at least one of these receptors. This invention describes the use of a substance as the active substance for the preparation of a composition in order to encourage at least one interaction of one chosen neuromediator among alpha-MSH, ACTH, and beta-endorphin, with their respective receptors.

The selected actives have been incorporated into cosmetic, neutraceutic, and pharmaceutical and preferably dermatological compositions, in particular, for application in the prevention and/or fight against the diminishing of epidermal thickness, notably during chronobiological or photo-induced cutaneous aging.

Advantageously, the aforementioned substance increases or stimulates the expression of a gene coding a receptor of a neuromediator coded by the POMC gene to fight against or prevent a deregulation of the balance between ligand and receptor in keratinocytes and/or in nervous cells.

Advantageously, the aforementioned substances diminish the expression of the POMC gene to fight against or prevent a deregulation of the expressions of ligands and receptors respectively observed in keratinocytes and/or nervous cells in particular during aging.

Advantageously, the substances can be used in combination in order to obtain inhibition of the expression of the POMC gene and stimulation of the expression of the receptors gene.

Advantageously the aforementioned substances increase or maintain the expression of the POMC gene to increase aforementioned effects of alpha-MSH, ACTH, and beta-endorphin.

Advantageously, the said substance increases the expression of at least one receptor of a neuromediator coded by the POMC gene in keratinocytes and/or nervous cells, to prevent and/or fight against cutaneous aging or the effects of stress inducing variations such as those observed during cutaneous aging.

Thus this invention concerns the modulation, preferably stimulation of these receptors in particular to palliate a deregulation, notably a decreasing in the interaction of alpha-MSH, and/or ACTH, and/or beta-endorphin with their respective receptors, linked to cellular aging and affecting keratinocytes and/or nervous cells, including cutaneous nervous network.

Advantageously, the said substance increases the synthesis of NGF, and thus potentially increases the growth of peripheral nerves at the cutaneous level. Thus this invention concerns the use of a substance permitting the increase of cutaneous innervation, observed to diminish during aging or under the effect of stress inducing variations such as those observed during the process of cutaneous aging.

Advantageously, the said substance increases the synthesis of VEGF, and thus potentially increases vascularization at the cutaneous level. Thus this invention concerns the use of a substance permitting better cutaneous irrigation, which is observed to diminish during aging or under the effect of stress inducing variations such as those during the process of cutaneous aging.

Preferably, the substance stimulating the expression of MC-1R and/or MC-2R and/or µ opioid receptor in keratinocytes and/or nervous cells is chosen from among:
4 characterized molecules:
   - phenylacetic acid 3,6,9-trimethyl-2,7-dioxo-2,3,3a,4,5,7,9a,9b-octahydro-azuleno[4,5-b]furan-4-yl,
   - Z-decahydro-6,8a-dihydroxy-1-isopropyl-3a, 6-dimethylazulen-5-yl-2 methylbut-2-enoate
   - 2.6 dimethyl-4(2-methyl butenoate) 5-6 (1-isopropyl-1 hydroxycyclopentane)) 1,2 epoxycycloheptane under CAS number 352220-52-1 as well as esters thereof
   - 7-acetate-2.6 dimethyl-4(2-methyl butenoate) 5-6 (1-isopropyl-1 hydroxycyclopentane)) 1,2 epoxycycloheptane known under CAS number 86992-41-8 and known under common name lapiferrin
   As well as vegetal extracts containing such characterized molecules.
6 plant extracts: galeopsis ochroleuca (wild hemp), Achillea millefolium (common yarrow), Colocasia esculenta (wild taro), Prunus cerasus (sour cherry), Prunus spinosa (blackthorn), Extract of Eburnamonine *(Apocynacea Amsonia Abrenaemontan);*
4 biotechnological hydrolysates obtained via fermentation of plant extracts with microorganisms present: extract of mango, date, or papaya, transformed by lactic bacteria, in particular lactobacillus, for example by lactobacillus plantarum, or banana extract transformed by lactic bacteria, in particular lactobacillus, for example by lactobacillus paracasei, and any mixture of these.

Among characterized molecules, lapiferin, notably in and/or from Ferula lapidosa extract, and phenylacetic acid 3,6,9-trimethyl-2,7-dioxo-2,3,3a,4,5,7,9a,9b-octahydro-azuleno[4,5-b]furan-4-yl are preferred substances according to the invention.

Among plants extracts, Achillea millefolium (common yarrow) and Prunus spinosa (blackthorn) are preferred substances according to the invention.

According to a preferred embodiement, the plant extracts are preferably obtained by macerating plants (preferably roots, rhizomes, stems, bark, flowers, fruits, seeds, germs, or leaves) at 1-10 % (p/p), usually 1-5 % in a solvent or a mixture of solvents, usually a mixture of water and alcohol, glycol, or polyol (such as ethanol, glycerol, butylene glycol and other glycols, xylitol, etc.) 100/0 to 0/100, and preferably in water. The extracts obtained are eventually then filtered or distilled in order to recover the soluble portion, which is then filtered, preferably at 0.45µm. Biotechnological hydrolysates are obtained via the fermentation of plant extracts in the presence of microorganisms usually from the family of lactic bacteria, in particular bactobacillus plantarum, or paracasei, and bifidobacterium, in particular longum or Saccharomyces. These hydrolysates are preferably then further filtered, preferably to 0.45µm, in order to obtain the active components.

The characterized molecules including lapiferin and phenylacetic acid 3,6,9-trimethyl-2,7-dioxo-2,3,3a,4,5,7,9a,9b-octahydro-azuleno[4,5-b]furan-4-yl are prepared via dissolution in a solvent, usually DMSO, ethanol, glycol, and in particular DMSO at a concentration of 0.1%.

The aforementioned active substances are preferably used in cosmetic compositions in an amount comprised between 0.01% and 5% (v/v) for plant extracts and biotechnological hydrolysates and in an amount comprised from 1.10⁻⁷% and 1% (v/v) for characterized molecules and preferably between 1.10⁻⁵ % and 1.10⁻¹%.

The aforementioned substances have shown also stimulating effects on the expression of MC-1R and/or MC-2R and/or µ opioid receptor in nerves cells and in particular in cutaneous nervous network.

This invention particularly concerns a composition intended for cosmetic care or prophylactic or therapeutic treatment in order to improve hyperpermeability during skin scarring, improve cell proliferation and differentiation, notably at the epidermal level, thus improving epithelialization of the skin and thus encouraging maintenance of skin innervation.

This invention concerns also composition intended for prophylactic or therapeutic treatment in order to improve innervation, to prevent and/or fight against pathologies due to, associated with and/or inducing lost of number and/or activity of nervous cells, notably in case of scaring and/or wound healing, stretchmarks, burns, psoriasis or dermatitis.

The aforementioned active substance as well as those thereafter described modulating expression of MC-1R and/or µ opioid R is preferably used in combination with another active substance chosen from among the group consisting of:
- active components stimulating the trophicity of cutaneous nerves and/or activating sensitive cutaneous nerves, like for example those cited in the patent application FR 2825273, extract of paprika (Capsicum annuum), red pigment (Red pepper), or pepper (Piper nigrum), or glutamylamidoethylindole (Exsymol);
- the active components imitating the effect of beta endorphin in order to improve the barrier function of the skin, like for example those cited in patent application US 2006069032, extract of cocoa bean hull;
- The active components stimulating the synthesis of beta endorphin in order to give a sensation of well-being, for example Tephroline, from the plant tephrosia purpurea (Soliance);
- The active components stimulating cellular proliferation and/or cellular differentiation, intended to have anti-aging activity, such as the following molecules: NGF, α-MSH, β endorphin, or derivatives, for example those described in patent application FR 2857874, P3-endorphin.
- active components protecting Fibroblast Growth Factor against degradation, notably FGF2 such as vegetal extract described in patent application filed by Applicant published under GB244036 in particular Hibiscus Abelmoscus extract
- active components stimulating activity and/or proliferation of fibroblasts such as a fermented peptide of soy like a product marketed by the Applicatant under the tradename Phytokine ^{™} , optionally in combination with Hibiscus Abelmoscus extract
- active components stimulating Hyaluronase synthase, notably Hyaluronase synthase 2 as described in patent FR2893252,
- active components stimulating activity and/or synthesis of Lysyl Oxidase like LOXL such as components described in patent number FR2855968 in particular dill extract for stimulation of elastic fibers.
- active components with anti-inflammatory properties such as those inhibiting PLA2, and in particular components described in patent FR2847267 and notably an extract of pueraria lobata roots (Inhipase®);
- active substances permitting a change in the color of the skin, such as active components for skin lightening and/or whitening;
- active substances with draining properties, like hesperitine laurate (Flavagrum®), or quercitine caprylate (Flavenger®).

According to a second aspect of the invention, the inventors identified active substances which modulate, preferably increase or decrease the expression of at least one receptor chosen from among MC-1R and µ opioid receptor expressed by melanocytes, and/or modulates the expression of the POMC gene expressed by melanocytes. This embodiement is particularly interesting in order to improve the color and/or tint of the skin and/or modify skin pigmentation, notably when the pigmentation presents localized flaws, such as pigmentary spots, to prevent and/or fight against pigmentation or depigmentation in particular pigmentary spots related or unrelated to aging as described thereafter.

The inventors have also developed screening process in melanocytes permitting to search for actives, notably among plant extracts or characterized chemical molecules or molecules transformed by technology, modulating in particular the expression of mRNAs coding MC-1R and µ opioid receptor, and possibly modulating the expression of mRNAs coding neuromediators coded by the POMC gene. The such selected actives have then been tested on the expression of proteins corresponding to the mRNAs.

The actives selected have been incorporated into cosmetic, neutraceutic, and pharmaceutical and preferably dermatological formulations, in particular for applications in the prevention and/or fight against pigmentation or depigmentation.

Advantageously, the aforementioned substance modulates the expression of at least one receptor of a neuromediator coded by the POMC gene in melanocytes to prevent or fight against pigmentation or depigmentation.

Advantageously, the aforementioned substance modulates the expression of the POMC gene to prevent or fight against pigmentation or depigmentation; in particular, substances simultaneously stimulating POMC and receptors in melanocytes are retained to increase pigmentation and the substances simultaneously decreasing or inhibiting POMC and receptors in melanocytes are retained to diminish pigmentation. The substances can be used in combination in order to obtain the stimulation of POMC expression and/or receptors expression and to increase pigmentation or in order to inhibit POMC and receptors and diminish pigmentation.

According to one embodiment, the substance stimulating the expression of at least one receptor chosen between MC-1R and/or µ opioid receptor and/or stimulating the expression of POMC in melanocytes is chosen from among:
- 1 characterized molecules: 1 methyl beta carbolin 3 carboxylic acid;
- Plant extracts from among the following: soy flour, (red colored) salsaparilla (Smilax ornata), Achillea millefolium (common yarrow), Cecropia obtusa, Oenothera biennis (evening primrose), carrot, male fern, Prunus cerasus (sour cherry), or any mixture of these, and;
- biotechnological hydrolysate obtained via the fermentation of plant extracts in the presence of microorganisms, chosen from among the following: fermented yeast or lupin, and in particular by lactic bacteria, such as for example by lactobacillus plantarum, papaya, date, or soy extract transformed by lactic bacteria, and in particular by lactobacillus, as for example by lactobacillus plantarum, lemon extract fermented by bifidobacterium, such as, for example, bifidobacterium longum, and any mixture of these.

Preferably, active substance is chosen among those stimulating MC-1R receptor and/or stimulating the expression of POMC in melanocytes, preferably :
- 1 characterized molecules: 1 methyl beta carbolin 3 carboxylic acid;
- Plant extracts from among the following: soy flour, Oenothera biennis (evening primrose), Salsaparilla (Smilax ornata), Cecropia obtusia, Achillea millefolium (common yarrow), Carrot, Prunus cerasus (sour cherry).
- biotechnological hydrolysate obtained via the fermentation of plant extracts in the presence of microorganisms, chosen from among the following : Papaya, lupin, yeast, date or soy extract transformed by lactic bacteria, and in particular by lactobacillus, as for example by lactobacillus plantarum.

This composition is particularly interesting to increase skin pigmentation, for tanning effect, to prevent and/or fight against depigmentation spots related or unrelated to aging.

According to second embodiment, the substance decreasing POMC gene in melanocytes is chosen between Galeopsis ochroleuca (wild hemp) and aqueous or hydroalcoolic extract of Juniperus communis (juniper berry) and/or the substance decreasing the expression of at least a receptor chosen between MC-1R and/or µ opioid receptor in melanocytes is aqueous or hydroalcoolic extract of Juniperus communis (juniper berry).

This composition is particularly interesting to decrease skin pigmentation, for whitening or lightening effect, to increase brightness of tint, to prevent and/or fight against pigmentation spots related or unrelated to aging.

This invention particularly concerns a composition intended for cosmetic or dermo-cosmetic care or prophylactic or therapeutic treatment in order to modify brightness of tint and pigmentation of the skin, and in particular pigmentary spots related or unrelated to aging.

The compounds in the present invention are prepared in the form of topical compositions, notably cosmetic, or pharmaceutical preferably dermatological compositions that are dermatologically acceptable. Thus, for these compositions, the excipient contains, for example, at least one compound chosen from among the group consisting of preservatives, emollients, emulsifiers, surfactants, moisturizers, thickeners, conditioners, mattifying agents, stabilizers, antioxidants, texturizing agents, shine agents, filmogenic agents, solubilizers, pigments, colorants, perfumes, and solar filters. These excipients are preferably chosen from among the group consisting of amino acids and their derivatives, polyglycerols, esters, polymers and cellulose derivatives, lanoline derivatives, phospholipids, lactoferrins, lactoperoxidases, sucrose-based stabilizers, vitamin E and its derivatives, natural and synthetic waxes, vegetable oils, triglycerides, insaponifiables, phytosterols, plant esters, silicones and their derivatives, protein hydrolysates, jojoba oil and its derivatives, lipo/hydrosoluble esters, betaines, aminoxides, saccharose ester plant extracts, titanium dioxides, glycines, parabens, and even more preferably from among the group consisting of butylene glycol, steareth-2, steareth-21, glycol-15 stearyl ether, cetearyl alcohol, phenoxyethanol, methylparaben, propylparaben, butylparaben, butylenes glycol, natural tocopherols, glycerine, dihydroxycetyl sodium phosphate, isopropyl hydroxycetyl ether, le glycol stearate, triisononanoin, octyl cocoate, polyacrylamide, isoparaffin, laureth-7, carbomer, propylene glycol, glycerol, bisabolol, dimethicone, sodium hydroxide, PEG 30-dipolyhydroxysterate, capric/caprylic triglycerides, cetearyl octanoate, dibutyl adipate, grapeseed oil, jojoba oil, magnesium sulfate, EDTA, cyclomethicone, xanthan gum, citric acid, sodium lauryl sulfate, mineral waxes and oils, isostearyl isostearate, dipelargonate of propylene glycol, isostearate of propylene glycol, PEG 8, beeswax, glyceride of hydrogenated palm kernel oil, lanolin oil, sesame oil, cetyl lactate, lanolin alcohol, titanium dioxide, lactose, saccharose, low-density polyethylene, and isotonic salt solution.

Ideally, the aforementioned compounds are formulated in a form chosen from among the group consisting of an aqueous or oil-based solution, a water-based crème or gel or an oily gel, usually in a jar or a tube, particularly a shower gel, shampoo, milk, emulsion, microemulsion or nanoemulsion, particularly oil-in-water or water-in-oil or multiple of silicone-based; a lotion, particularly in a glass or plastic bottle of a spray or aerosol bottle, a blister-pack, liquid soap, a dermatological bar of soap, a pomade, mousse, an anhydrous product, preferably liquid, cream or solid, for example in the form of a stick, particularly in the form of lipstick.

The term "topical application" used here means to apply or spray the composition of this invention onto the surface of the skin.

The term "dermatologically acceptable" used here means that the compound or compounds used are adapted for use in contact with human skin without undue toxicity, incompatibility, instability, allergic response, or their equivalents.

Numerous active cosmetic ingredients are known by professionals to improve the health and/or physical appearance of the skin. Professionals know how to formulate cosmetic or dermatological compositions to obtain the best effects. In addition, the compounds described in this invention can have a synergistic effect when they are combined with each other. These combinations are also covered by this invention. The CTFA Cosmetic Ingredient Handbook, Second Edition (1992) describes different cosmetic and pharmaceutical ingredients currently used in the cosmetic and pharmaceutical industry that are particularly adapted to topical use. Examples of these types of ingredients include but are not limited to the following compounds: abrasives, absorbent compounds, compounds with aesthetic purposes such as perfumes, pigments, colorants, essential oils, astringents, etc. (for example: clove oil, menthol, camphor, eucalyptus oil, eugenol, menthyl lactate, and hamelis distillate), anti-acne agents, anti-flocculant agents, anti-foaming agents, anti-microbial agents (for example iodopropyl butylcarbamate), les antioxidants, bonding agents, biological additives, tampon agents, swelling agents, chelatants, additives, biocidal agents, denaturants, external analgesics, film-forming materials, polymers, opacifying agents, pH adjusters, reducing agents, depigmenting or lightening agents (for example: hydroquinone, kojic acid, ascorbic acid, magnesium ascorbyl phosphate, ascorbyl glucosamine), conditioning agents (for example: humectants), calming agents for the skin and/or scarring agents (for example: panthenol and its derivatives, for example ethyl panthenol), aloe vera, pantothenic acid and its derivatives, allantoin, bisabolol and dipotassium glycyrrhizinate), thickeners, vitamins, and the derivatives or equivalents of these.

In the figures:
Figure 1 shows a significant decrease in the expression of the genes of receptors MC-1R, MC-2R and and µ opioid receptor on human biopsies in keratinocytes during aging process.
Figure 2 shows a significant decrease in the expression of tubulin βIII and the 200 neurofilaments via a method of immunofluorescence on biopsy sections of normal human skin.
Figures 3 and 4 represent the relative rate of expression of tubulin βIII and 200 neurofilaments respectively in subjects aged between 15 and 75 years.
Figures 5 and 6 represent the statistical analysis corresponding to the studies presented in figures 3 and 4 respectively.

Other goals, characteristics, and benefits of the invention will appear clearly to professionals upon reading of the explanatory description which refers to examples given solely for the purposes of illustration and should not in any way limit the scope of the invention.

The examples are an integral part of this invention and any characteristic that appears new compared to any previous technique from the description taken in its entirety, including examples, is an integral part of the invention in its function and generality.

Thus, each example has a general scope.

### EXAMPLES

### Example 1: Demonstration of a drop in the level of expression of the proteins MC-1R, MC-2R, and µ opioid receptor on human biopsies through immunohistology during the aging process

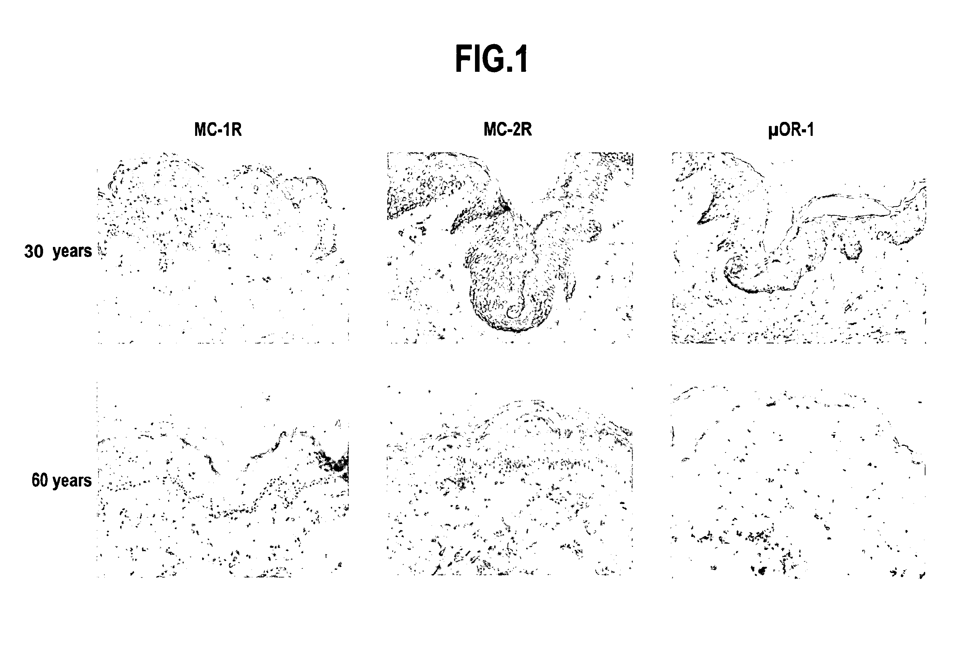

The inventors have demonstrated a variation of expression of the MC-1R, MC-2R, and µ opioid receptors at the proteic level during the process of chronobiological aging in biopsies taken from young women (younger than 40 years of age) and aged women (older than 50 years of age). (see figure 1 : Receptors expression in human biopsies form human patients aged 30 or 60 years old -immunohistochemistry results)

This study was implemented using an immunohistology study.

This demonstration was obtained without ambiguity thanks to the use of the antibodies anti-MC-1R (1/500), anti-MC-2R (1/200) and the anti- µ opioid receptor (1/1000).

The reconstructed skin model (Mimeskin®, Coletica, Lyon, France) and biopsies were prepared for immunomarkings in frozen cross-sections or after sealing in paraffin.

The antibodies used are the following: anti-MC-1R, anti-MC-2R, and anti-µ opioid receptor (Chemicon International). Detection is conducted with an appropriate conjugate secondary antibody.

Results shown in Figure 1 prove that expression at the level of the genes of the receptors MC-1R, MC-2R, and µ opioid R decreases in keratinocytes with aging.

### Example 2: Demonstration of a drop in the level of expression of the genes MC-1R, MC-2R, and µ opioid receptor and of an increase in the expression of the POMC gene in adult keratinocytes during the aging process via the RT-PCR method in real time

The invention also deals with the variation of expression of the genes of the receptors MC-1R, MC-2R, and µ opioid R and the variation of expression of the POMC gene during the process of chronobiological aging in keratinocytes taken from human biopsies. The expression of the four genes of interest as well as of the actin has been analyzed by RT-PCR in real time (reverse transcriptase polymerase quantitative chain reaction). This technique permits precise quantification of the expression of a gene by connecting it to that of the actin (considered as a constant). The regulation of the level of expression of this gene can thus be quantified.

The total RNA is purified with the "SV 96Total RNA Isolation System" kit (Promega, Charbonnières, France). The purified RNA is suspended in 100 µl of RNase-free water (Promega, Charbonnières, France), measured and divided into plates (96-well microtiter plates, 10µl of total RNA at 5ng/µl per PCR). The starters selected for use in this project are the following and the subject of table I:

**Table I:**

| | |
|---|---|
| Actin sense (SEQ ID N°5) | GTGGGGCGCCCCAGGCACCA |
| Actin antisense (SEQ ID N°6) | CTCCTTAATGTCACGCACGATTTC |
| MC-1R sense (SEQ ID N°7) | GGGCTCTGAGAACGACTTTT |
| MC-1R Antisense (SEQ ID N°8) | CCGGGCTCCTGTCTGGTTGG |
| MC-2R sense (SEQ ID N°9) | TCACGTCGCTGTTCCCGCTGAT |
| MC-2R Antisense (SEQ ID N°10) | AAGAGAGACATGTAGCAGGCGCAGTA |
| µ opioid receptor sense (SEQ ID N°11) | CTCAGCCAGGACTGGTTTCTGTAAGA |
| µ opioid receptor antisense (SEQ ID N°12) | TCGGACAGGTTGCCATCTAAGTG |
| POMC sense (SEQ ID N°13) | CGCCCAGTGAAGGTGTACCC |
| POMC Antisense (SEQ ID N°14) | GGCGTCTGGCTCTTCTCGGAGGTC |

The technique of RT-PCR in real time is conducted with the "Quanti Tect SYBR Green RT-PCR" kit (Qiagen, France) on plates containing mRNA, in an OPTICON thermocycler, which carries out cycles of amplification. Retrotranscription (RT) takes place for 30 minutes at 50°C, followed by 15 minutes at 95°C to inhibit reverse transcriptase, activate polymerase, and alter the complementary DNA (cDNA) obtained. 50 cycles of polymerization in sequence (PCR) are carried out (15 seconds at 95°C, 30 seconds at 60°C, 30 seconds at 72°C). At the end of each cycle, fluorescence, which is proportional to the number of fragments, is read. The level of expression is defined by the level of expression of each gene in comparison to actin.

The inventors have shown that the expression of the MC-1R gene is 4 times less expressed in women aged more than 52 years compared to young woman, and this in a statistically significant way.

The inventors have shown that the expression of the MC-2R gene is 8 times less expressed in women more than 5 years of age compared to young women, and this in a statistically significant way.

The inventors have shown that the expression of the gene of the µ opioid receptor is 6.5 times less expressed in women aged more than 55 years compared to young women, and this in a statistically significant way.

The inventors have shown that the expression of the POMC gene is 5 times more expressed in women aged more than 50 years compared to young women, and this in a statistically significant way.

In conclusion, the invention permits the demonstration of a disturbance of the balance between the expression of receptors and their ligand in human keratinocytes in favor of an increase of the ligand and a diminishing of all receptors.

### Example 3: Analysis of the expression of the RNA messengers of the receptors MC-1R, MC-2R and µ opioid R and of the mRNAs of POMC, for example via quantitative RT-PCR with or without the putting in contact of active components with keratinocytes

The invention particularly concerns a method of screening new molecules capable of inducing the synthesis of the 3 receptors MC-1R, MC-2R, and/or µ opioid R with the objective of restoring a physiological expression, such as found in the keratinocytes of young skin, of the receptors noted in aged keratinocytes in reconstructed skin, biopsies of human skin.

The active components are of different origins (plant or synthetic molecules, for example).

In particular, the extracts are obtained by macerating plants (preferably roots, rhizomes, stems, bark, flowers, fruits, seeds, germs, or leaves) at 1-10 % (p/p), usually 1-5 % in a solvent or a mixture of solvents, usually a mixture of water and alcohol, glycol, or polyol (such as ethanol, glycerol, butylene glycol and other glycols, xylitol, etc.) 100/0 to 0/100, and preferably in water. The extracts obtained are then filtered or distilled in order to recover the soluble portion, which is then filtered, preferably at 0.45µm. Biotechnological hydrolysates are obtained via the fermentation of plant extracts in the presence of microorganisms usually from the family of lactic bacteria, in particular bactobacillus plantarum, or paracasei, and bifidobacterium, in particular longum or Saccharomyces. These hydrolysates are then filtered, preferably to 0.45µm, in order to obtain the active components.

The characterized molecules are prepared via dissolution in a solvent, usually DMSO, ethanol, glycol, and in particular DMSO at a concentration of 0.1%.

The active components are tested for keratinocytes after dissolution in the culturing environment: usually between 0.1% and 2% (v/v) for plants and biotechnological hydrolysates and in particular from 0.5 % to 1% and usually between 0.001% and 0.01% for characterized molecules. The present results are obtained with 1% in the culturing environment for plant extracts and biotechnological hydrolysates, and with 0.01% for characterized molecules.

Keratinocytes taken from plastic surgery on normal adults through enzymatic digestion have been amplified in a specific environment for keratinocytes and then seeded, for example at 50,000 per cm² in 24-well plates and cultivated in a single layer in a definite no-serum environment. Upon convergence, the cells are put in contact with diluted actives in the culturing environment, usually for 24 hours.

In parallel, an untreated control (environment alone) and 3 positive controls (TGF-β at 1ng/ml, IL-1α at 50 pg/ml and TNF-α at 100 ng/mL) are usually carried out, for example on the same cultivation plate.
TGF-β at 1ng/ml, TNF-α at 100 ng/mL, and IL-1α at 50 pg/ml were previously tested and stimulation of mRNA synthesis of the 3 receptors induced by these 3 cytokins at these concentrations was verified through an analysis of mRNA, for example by quantatitive RT-PCR (x2 or x.08 for the 3 inducers).

After the contact time of the actives with the cells, for example 24 hours, the environments are eliminated and the cells conserved, for example by freeze-drying at-80°C after a rinsing with 7.4 pH phosphate solution. The total RNA is extracted, for example, using a 96-well extraction kit on silica columns and divided onto a 96-well spectrophotometer at 260 nm (purity indicator: dosage of proteins at 280 nm). The RNA is diluted, for example at 5ng/µl. Qualitative RT-PCR in 1 stage is carried out, for example, on 50 ng of initial RNA on a 96-well plate, on genes of actin, MC-1R, MC-2R, µ opioid R, and POMC. The specific primers of each gene are used, for example at 0.5µM, specified in table I above.

The parameters of amplification have usually been the following: The technique of RT-PCR in real time is conducted with the "Quanti Tect SYBR Green RT-PCR" kit (Qiagen, France) on wells containing mRNA, in an OPTICON thermocycler, which carries out cycles of amplification. Retrotranscription (RT) takes place for 30 minutes at 50°C, followed by 15 minutes at 95°C to inhibit reverse transcriptase, activate polymerase, and alter the complementary DNA (cDNA) obtained. 50 cycles of polymerization in sequence (PCR) are carried out (15 seconds at 95°C, 30 seconds at 60°C, and 30 seconds at 72°C). At the end of each cycle, fluorescence, which is proportional to the number of fragments amplified, is read. The level of expression is defined by comparing the expression of each gene to actin.

The level of expression of the genes MC-1R, MC-2R, µ opioid R, and POMC have been expressed in percentage of variation in comparison with those obtained for the negative control (without treatment). These actives are the following and are the subject of table II.

**Table II**

| **Name (Latin)** | **Part of the plant** | **MC-1R Control X:** | **MC-2R Control X:** | **µ opioid R Control X:** | **POMC Control X:** |
|---|---|---|---|---|---|
| Achillea millefolium | Plant | 2.73 | 3.0 | 23.0 | 3.38 |
| Galeopsis ochroleuca | plant | 2.9 | 1.0 | 1.0 | 1 |
| Colocasia esculenta | tube | 2.0 | 1.0 | 24.0 | 1 |
| Prunus cerasus | stem | 1.0 | 1.0 | 5.3 | 2.6 |
| Oenothera biennis | seed | 0.67 | 1.0 | 1.0 | 0.36 |
| Prunus spinosa | fruit | 1.0 | 2.3 | 6.0 | 1 |
| (Z)-decahydro-6 ,8a-dihydroxy-1 -isopropyl-3a, 6-dimethylazulen-5-yl 2-methylbut-2-enoate | molecule | 1.0 | 18.5 | 1.0 | 1 |
| Phenyl-acetic acid 3,6,9-trimethyl-2,7-dioxo-2,3,3a,4,5,7,9a,9b -octahydro-azuleno[4,5-b]furan-4-yl | molecule | 20.0 | 20.0 | 1.0 | 1 |
| Mango Lactobacillus plantarum | Fruit | 1.0 | 10 | 3 | 0.4 |
| Date lactobacillus Planctarum | Fruit | 1.0 | 1 | 60 | 1 |
| Papaya lactic bacteria | Fruit | 1.1 | 4 | 5 | 0.6 |
| Banana lactic bacteria | Fruit | 1.2 | 10 | 42 | 0.4 |
| Lapiferin | molecule | 1.0 | 2.8 | 4.0 | 1.0 |

| | | | | | |
|---|---|---|---|---|---|
| "Control X" means "control multiplied by" | | | | | |

Conclusion: 13 actives among 960 are capable of significantly increasing, under the conditions considered, the rates of mRNA in genes coding MC-1R or MC-2R or µ opioid R in keratinocytes. In addition, 4 actives in particular inhibit the expression of the POMC gene.

### Example 4: Analysis of the expression of the RNA messengers of the receptors MC-1R and µ opioid R, for example via quantitative RT-PCR with or without putting in contact of active components with melanocytes

The experimentation is carried out under the same conditions described in the preceding example except for the cellular cultivation.

Melanocytes extracted from plastic surgery in normal adults via enzymatic digestion have been amplified in a specific environment for melanocytes; the melanocytes were then seeded, for example at 50,000 per cm² in 24-well plates and cultivated in an environment specific for melanocytes. Upon convergence, the cells are put in contact with diluted actives in the cultivation environment, usually for 24 hours.

**Table III**

| **Name (Latin)** | **Part of the plant** | **MC-1R Control X:** | **µ opioid R Control X :** | **POMC Control X:** |
|---|---|---|---|---|
| Achillea millefolium | Plant | 1.0 | 1.0 | 4.3 |
| Galeopsis ochroleuca | Plant | 1.0 | 1.0 | 0.47 |
| Oenothera biennis | seed | 4.0 | 1.0 | 1.96 |
| 1 methyl beta carbolin-3-carboxylic acid | | 1.0 | 1.0 | 3.3 |
| Yeast Lactic bacteria | | 1.0 | 1.0 | 2.18 |
| Lemon bifidobacterium longum | | 1.0 | 1.0 | 2.18 |
| Salsaparilla (Smilax ornata) | root | 3.9 | 1.0 | 2.74 |
| Juniperus communis | fruit | 0.4 | 1.0 | 0.25 |
| Cecropia obtusia | buds | 3.2 | 1.0 | 2.18 |
| Papaya lactic bacteria | | 2.58 | 1.0 | 1.0 |
| Carrot | fruit | 1.97 | 1.0 | 1.0 |
| Sour cherry | fruit | 2.0 | 1.0 | 1.0 |
| Lupin lactobacillus plantarum | | 1.0 | 1.0 | 2.15 |
| Soy flour | // | 1.0 | 1.0 | 2.42 |
| Date lactobacillus plantarum | fruit | 3.23 | 1.0 | 1.0 |
| Soy lactobacillus plantarum | | 2.0 | 1.0 | 1.0 |

| | | | | |
|---|---|---|---|---|
| "Control X" means "control multiplied by" | | | | |

Conclusion: 10 actives increase the expression of the MC-1R gene in melanocytes; 9 actives increase the expression of the POMC gene, and 2 actives diminish the expression of the POMC gene in melanocytes.

### Example 5: Detection of MC-1R, MC-2R and µ opioid receptor proteins in keratinocytes after action of active components

Electrophoresis shows the characterization of the proteins MC-1R, MC-2R, and µ opioid receptor thanks to the following respective commercial polyclonal antibodies: OPA1-15013 (Affinity bioreagents), AB5128 (Chemicon International), and AB5511 (Chemicon International).

The cells were cultivated at 37°C in an atmosphere of 5% CO₂ in the K-SFM environment K-SFM (Invitrogen) containing BPE (25 mg), EGF (2.5 µg), and normocin.

The cells were washed once with PBS solution, and the proteins were extracted at 30 min at 4°C in lysis solution (Tris 50 mM, NaCl 250 mM, pH 7.5, 1% triton), in the presence of protease inhibitors. The lysates were centrifuged for 15 minutes at 13,000 g. The floaters are diluted with Laemmli solution in the presence of beta mercaptoethanol before electrophoresis.

For immunodetection, the proteins are separated via electrophoresis in 4-12% SDS-polyacrylamide gel. The proteins were transferred onto a nitrocellulose membrane (Biorad). The membranes are then saturated for one hour at ambient temperature in TBS solution with 3% BSA. The proteins are finally immunodetected after incubation of the primary antibody at 4°C all night, followed by the secondary antibody coupled with Alexa 488 (invitrogen), 1 hour at ambient temperature.

The antibodies were used in the following dilution: 5 µg/mL anti-MC-1R, 5 µg/mL anti-MC-2R, and 1/1000 anti-µ opioid R.

Semi-quantification was carried out via image analysis. The actives are the following and are the subject of table IV.

**Table IV**

| **Name** | **Part of the plant:** | **MC-2R Control X:** | **µ opioid R Control X:** |
|---|---|---|---|
| Achillea millefolium | Plant | 18.2 | 2.81 |
| Galeopsis ochroleuca | Plant | 1.0 | 2.54 |
| Colocasia esculenta | tuber | 1.0 | 1.52 |
| Prunus cerasus | Stem | 1.0 | 1.44 |
| Oenothera biennis | seed | 1.0 | 1.46 |
| Prunus spinosa | fruit | 8.26 | 2.02 |
| (Z)-decahydro-6 ,8a-dihydroxy-1 -isopropyl-3a, 6-dimethylazulen-5-yl 2-methylbut-2-enoate | | 1.99 | 1.37 |
| Phenyl-acetic acid 3,6,9-trimethyl-2,7 | | 1.96 | 1.66 |
| -dioxo-2,3,3a,4,5,7,9a,9b-octahydro-azuleno[4,5-b]furan-4-yl | | | |

| | | | |
|---|---|---|---|
| "Control X" means "control multiplied by" | | | |

Conclusion: The increases induced by the actives observed at gene level are confirmed by increases at the protein level. Galeopsis ochroleuca, Oenothera biennis and the two characterized molecules induce an increase in the expression of the protein µ opioid R though the expression of the gene is not increased.

### Example 6: Immunodetection of βIII tubulin and neurofilament 200 in human biopsies

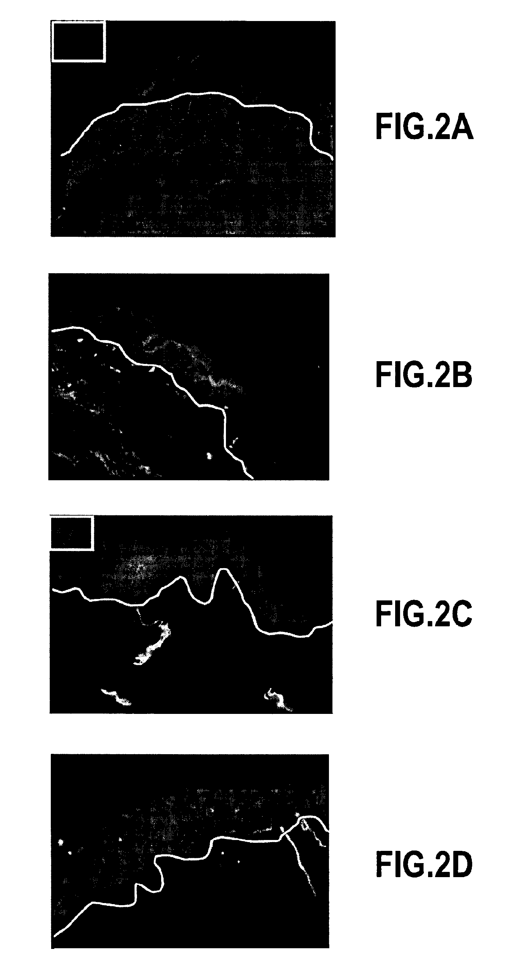

The inventors have shown that during the process of chronobiological aging, the expression of tubulin βIII and neurofilament 200 were diminished during the neurobiological aging process by immunofluorescence (fig. 2). The inventors have chosen these two markers that permit the detection of the presence of axonal prolongations in the skin. The marker tubulin βIII permits a quantification of the number of cellular cores and the density of the neuritic network, and the marker neurofilament 200 is more indicative of a maturing of the neuritic network. From an immunofluorescence study carried out on 80 mammography biopsies of women aged between 17 and 75 years, the inventors have been able to show that the expression of tubulin βIII was diminished 3.7 times after 38 years and that the expression of neurofilaments 200 was diminished by 3.5 times. The antibodies were used in the following dilution: 1:500 (anti-tubulin βIII) and 1:500 (anti-neurofilament 200). The immune complexes are detected with an anti IgG of mice (goat) joined to ALEXA 594 diluted to 1:100.

In figure 2, the detection via immunofluorescence of tubulin βIII and of neurofilaments 200 in normal human skin is represented.

Immunodetection of tubulin βIII in a so-called young subject (26 years old) is indicated in A and the immunodetection of tubulin βIII in a so-called aged subject (56 years old) is indicated in B. Immunodetection of neurofilaments 200 in a so-called young subject (26 years old) is indicated in C and the immunodetection of neurofilaments 200 in a so-called aged subject (56 years old) is indicated in D. The position of the dermo-epidermal junction is indicated by the white line. The squares on the upper left of each figure indicate a negative control using a controlled isotypical antibody.

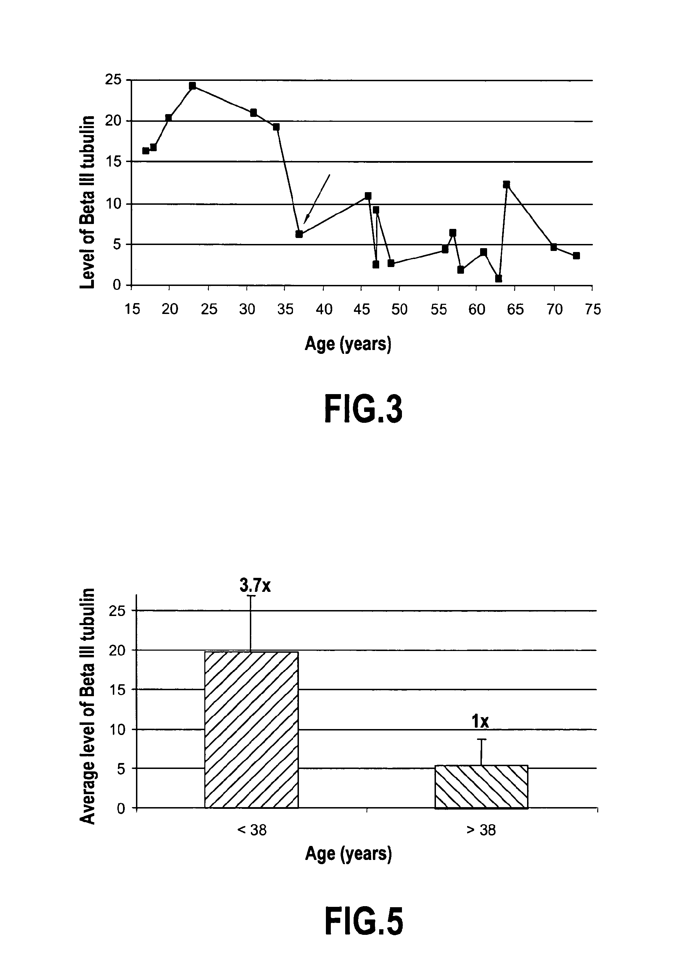

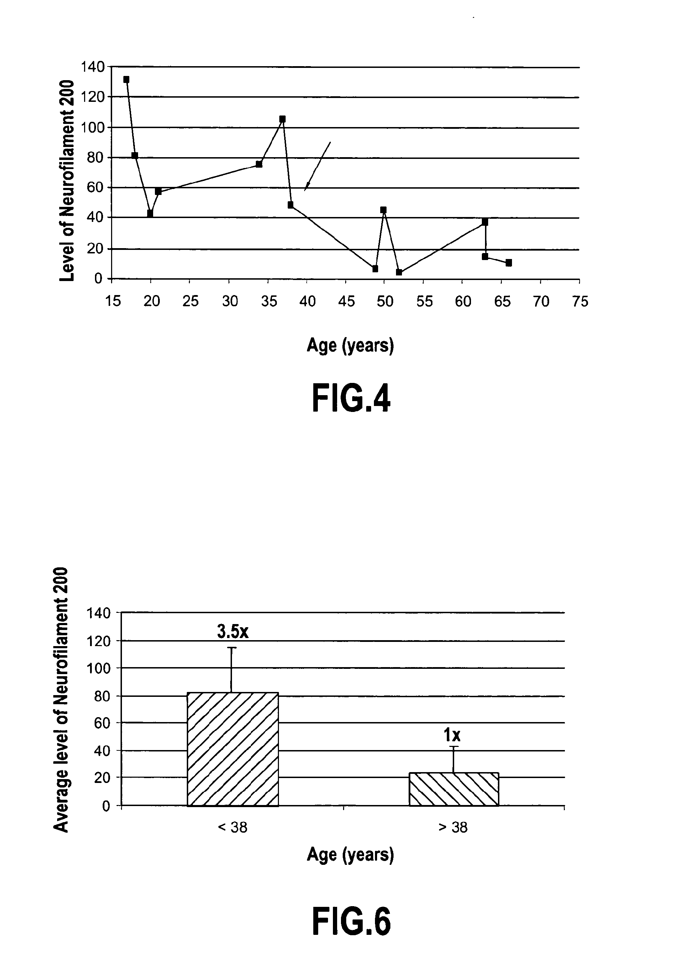

Figures 3 and 4 represent the relative rates of expression of tubulin βIII and of neurofilaments 200 respectively, in subjects aged between 15 and 75 years. The red arrow indicates the break in the curve.
Figures 5 and 6 represent the statistical analysis corresponding to the studies presented in figures 3 and 4, respectively. The results indicate that βIII tubulin and the neurofilaments 200 are 3.7 and 3.5 time (respectively) more expressed in subjects younger than 38 years of age. The results presented represent the average ± SD with a test t with P = 0.05.

### Example 7: Dosages of NGF and VEGF secreted in floaters in keratinocyte cultivation

Keratinocytes in aged subjects are put into cultivation, and after the time in contact of the actives with the cells, for example 24 hours, the environments are recovered and subjected to an ELISA technique permitting the detection of the secretion of NGF or VEGF. The method of use is in accordance with the protocol undergone by the R and D system provider (DY256) for NGF and Clinisciences (KHG0112) for VEGF. Among the actives tested, the following actives present the strongest modulation, in particular of NGF, and are the subject of table V.

**Table V**

| **Plant** | **Part of the plant** | **NGF control multiplied by** |
|---|---|---|
| Achillea millefolium | Plant | 11.28 |
| Prunus cerasus | Stem | 17.8 |
| Galeopsis ochroleuca | Plant | 11.24 |

### Example 8: Immunodetection of beta III tubulin and neurofilament 200 in surviving human biopsies

Biopsies of adult plastic surgery are maintained in survival for 4 days in an environment of DMEM cultivation, in the presence or not of NGF or of an active component. The biopsies are then frozen and subject to immunofluorescence studies for the identification of neuronal markers, in particular those indicated in example 6.

### Example 9: Immunodetection of differentiation and proliferation markers in models of reconstructed skin.

This model is the association of a cultivation of a reconstructed dermis on which the supplementary cultivation of a reconstructed epidermis is then carried out. The reconstructed dermis model is created according to the following protocol:
- 0.5 to 1.10⁶ normal human skin fibroblasts are seeded on a collagen-based substratum matrix, usually of glycosaminoglycane chitosan, and then cultivated in a nutritive environment, for example DMEM-Glutamax supplemented with 10% calf serum, ascorbic acid, and preferably at a final concentration of 1mM EGF (epidermal growth factor), and preferably at a final concentration of 10 ng/mL Normocin, and preferably at a final concentration of 100 µg/mL, for 21 days.

The reconstructed skin model is created according to the following protocol:
- 0.5 to 1.10⁶ normal human keratinocytes are seeded on the dermal equivalent, then cultivated in a nutritive environment, for example DMEM-Glutamax/Ham F-12 (ratio 3/1 v/v) supplemented with calf serum, ascorbic acid, and preferably at a final concentration of 1 mM, EGF *(epidermal growth factor)* and preferably at a final concentration of 10 ng/mL hydrocortisone and preferably at a final concentration of 0.4 µg/mL umuline and preferably at a final concentration of 0.12 UI/mL isuprel and preferably at a final concentration of 0.4 µg/mL triiodothyronine and preferably at a final concentration of 2.10⁻⁹ M adenine and preferably at a final concentration of 24.3 µg/mL Normocin and preferably at a final concentration of 100 µg/mL. Cultivation takes place for 7 days in immersed conditions. The cultures are then placed in an air-liquid interface for 14 additional days in the same environment as the culture in immersion, except for the calf serum, hydrocortisone, isuprel, triiodothyronine, and umuline.

The reconstructed skin (Mimeskin®, Coletica, Lyon, France) was then prepared in Boulin fixing solution (LOX, LOXL, elastin) or in a 10% formol solution (for elastin), then sealed in paraffin for an immunihistochemical study, or directly frozen in liquid nitrogen for analysis of immunofluorescence. 6 µm-thick sections were extracted from the paraffin and bleached in glycine-HCl (100 mmol/l). Immunodetections of Ki67 (proliferation marker), Keratin 14 (marker of all cellular layers), keratin 10 (marker of suprabasal keratinocyte layers), involucrine, tranglutaminase, and nestine were carried out on the reconstructed skin on day 45.

### Example 10: Use of products of the invention in combination with existing patented molecules

It is possible to combine products of the invention with, in particular, extracts stimulating the trophicity of cutaneous nerves active on sensitive cutaneous nerves, like for example paprika extract (Capsicum annuum) and/or red pigment (red pepper) and/or pepper (Piper nigrum) (L'OREAL FR2825273), and or glutamylamidoethylindole (Exsymol).

It is feasible to combine products of the invention with extracts that imitate the action of β endorphin in order to improve the barrier function of the skin, like for example extract of hull of cocoa bean (L'OREAL US2006069032).

It is also feasible to combine products of the invention with extracts that activate β endorphin in order to generate a feeling of well-being, like for example extract of Tephrosia purpurea (faux indigo).

It is also feasible to combine products of the invention with other molecules such as α-MSH, β endorphin, or derivatives such as, for example, P3-endorphin and essential oils in order to stimulate the differentiation of keratinocytes intended to have an anti-aging effect (CODIF FR2857874) of NGF in order to have an effect on nerve trophicity.

### Example 11: Use of products of the invention in cosmetic or pharmaceutical formulations of the oil-in-water emulsion type

### Formulation 11a:

| | | |
|---|---|---|
| A | Water | qsp 100 |
| | Butylene Glycol | 2 |
| | Glycerine | 3 |
| | Sodium Dihydroxycetyl Phosphate, Isopropyl Hydroxycetyl Ether | 2 |
| | | |
| B | Glycol Stearate SE | 14 |
| | Triisononaoin | 5 |
| | Octyl Cocoate | 6 |
| | | |
| C | Butylene Glycol, Methylparaben, Ethylparaben, Propylparaben, pH adjusted to 5.5 | 2 |
| | | |
| D | Products of the invention | 0.01-10% |

### Formulation 11b:

| | | |
|---|---|---|
| A | Water | qsp 100 |
| | Butylene Glycol | 2 |
| | Glycerine | 3 |
| | Polyacrylamide, Isoparafin, Laureth-7 | 2.8 |
| | | |
| B | Butylene Glycol, Methylparaben, Ethylparaben, Propylparaben; | 2 |
| | | |
| | Phenoxyethanol, Methylparaben, Propylparaben, Butylparaben, Ethylparaben | 2 |
| | | |
| | Butylene Glycol | 0.5 |
| | | |
| D | Products of the invention | 0.01-10% |

### Formulation 11c:

| | | |
|---|---|---|
| A | Carbomer | 0.50 |
| | Propylene Glycol | 3 |
| | Glycerol | 5 |
| | Water | qsp 100 |
| | | |
| B | Octyl Cocoate | 5 |
| | Bisabolol | 0.30 |
| | Dimethicone | 0.30 |
| | | |
| C | Sodium Hydroxide | 1.60 |
| | | |
| D | Phenoxyethanol, Methylparaben, Propylparaben, Butylparaben, Ethylparaben | 0.50 |
| | | |
| E | Perfume | 0.30 |
| | | |
| F | Products of the invention | 0.01-10% |

### Example 12: Use of products of the invention in a formulation of the water-in-oil type

| | | |
|---|---|---|
| A | PEG 30 - dipolyhydroxystearate | 3 |
| | Capric Triglycerides | 3 |
| | Cetearyl Octanoate | 4 |
| | Dibutyl Adipate | 3 |
| | Grape Seed Oil | 1.5 |
| | Jojoba Oil | 1.5 |
| | Phenoxyethanol, Methylparaben, Propylparaben, Butylparaben, Ethylparaben | 0.5 |
| | | |
| B | Glycerine | 3 |
| | Butylene Glycol | 3 |
| | Magnesium Sulfate | 0.5 |
| | EDTA | 0.05 |
| | Water | qsp 100 |
| | | |
| C | Cyclomethicone | 1 |
| | Dimethicone | 1 |
| | | |
| D | Perfume | 0.3 |
| | | |
| E | Products of the invention | 0.01-10% |

### Example 13: Use of products of the invention in a formulation of the shampoo or shower gel type

| | | |
|---|---|---|
| A | Xanthan Gum | 0.8 |
| | Water | qsp 100 |
| | | |
| B | Butylene Glycol, Methylparaben, Ethylparaben, Propylparaben | 0.5 |
| | Phenoxyethanol, Methylparaben, Propylparaben, Butylparaben, Ethylparaben | 0.5 |
| | | |
| C | Citric acid | 0.8 |
| D | Sodium Laureth Sulfate | 40.0 |
| | | |
| E | Products of the invention | 0.01-10% |

### Example 14: Use of products of the invention in a formulation of the lipstick type or other anhydrous products

| | | |
|---|---|---|
| A | Mineral Wax | 17.0 |
| | Isostearyl Isostearate | 31.5 |
| | Propylene Glycol Dipelargonate | 2.6 |
| | Propylene Glycol Isostearate | 1.7 |
| | PEG 8 Beeswax | 3.0 |
| | Hydrogenated Palm Kernel Oil Glycerides, Hydrogenated Palm Glycerides | 3.4 |
| | Lanoline Oil | 3.4 |
| | Sesame Oil | 1.7 |
| | Cetyl Lactate | 1.7 |
| | Mineral Oil, Lanolin Alcohol | 3.0 |
| | | |
| B | Castor Oil | qsp 100 |
| | Titanium Dioxide | 3.9 |
| | CI 15850:1 | 0.616 |
| | CI 45410:1 | 0.256 |
| | CI 19140:1 | 0.048 |
| | CI 77491 | 2.048 |
| | | |
| C | Products of the invention | 0.01-5% |

### Example 15: Use of products of the invention in a formulation of aqueous gels (eye contouring, slimming, etc.)

| | | |
|---|---|---|
| A | Water | qsp 100 |
| | Carbomer | 0.5 |
| | Butylene Glycol | 15 |
| | Phenoxyethanol, Methylparaben, Propylparaben, Butylparaben, Ethylparaben | 0.5 |
| | | |
| B | Products of the invention | 0.01-10% |

### Example 16: Use of products of the invention in a formulation of the triple-emulsion type

| Primary emulsion W1/O | | |
|---|---|---|
| A | PEG 30 - dipolyhydroxystearate | 4 |
| | Capric Triglycerides | 7.5 |
| | Isohexadecane | 15 |
| | PPG-15 Stearyl ether | 7.5 |
| | | |
| B | Water | 65.3 |
| | | |
| C | Phenoxyethanol, Methylparaben, Propylparaben, Butylparaben, Ethylparaben | 0.7 |

| Secondary emulsion W1/O/W2 | | |
|---|---|---|
| A | Primary emulsion | 60 |
| | | |
| B | Poloxamer 407 | 2 |
| | Phenoxyethanol, Methylparaben, Propylparaben,2-bromo-2nitropropane-1,3 diol | 0.3 |
| | Water | qsp 100 |
| | | |
| C | Carbomer | 15 |
| | | |
| D | Triethanolamine | PH 6.0-6.5 |

### Example 17: Preparation of pharmaceutical formulations containing the product of the invention

| | *Formulation 17 a: preparation of pills* | |
|---|---|---|
| A | Excipients | In g per pill |
| | Lactose | 0.359 |
| | Saccharose | 0.240 |
| | | |
| B | Products of the invention* | 0.001 -0.1 |

| | | |
|---|---|---|
| *The product of the invention is obtained, for example, according to the extraction procedure described in example 3 followed by a drying stage. | | |

| *Formulation 17 b: preparation of a pomade* | | |
|---|---|---|
| A | Excipients | |
| | Low-density polyethylene | 5.5 |
| | Liquid paraffin | qsp 100 |
| | | |
| B | Products of the invention* | 0.001 -0.1 |

| | | |
|---|---|---|
| *The product of the invention is obtained, for example, according to the extraction procedure described in example 3 followed by a drying stage. | | |

| *Formulation 17 c: preparation of an injectable formula* | | |
|---|---|---|
| A | Excipient | |
| | Salt isotonic solution | 5 ml |
| | | |
| B | Products of the invention* | 0.001 -0.1 g |

| | | |
|---|---|---|
| *The product of the invention is obtained, for example, according to the extraction procedure described in example 3 followed by a drying stage. | | |

It folllows a list of further embodiments of the invention
Embodiment 1. Use of at least one substance modulating the expression of MC-R in melanocytes, as an active ingredient in a cosmetic composition or for the preparation of a neutraceutical composition or for the preparation of a pharmaceutical composition, and notably a dermatological composition.
Embodiment 2. Use according to embodiment 1 to fight against and/or prevent a deregulation of the effect of alpha-MSH and/or ACTH.
Embodiment 3. Use according to one of the previous embodiments to prevent and/or fight against cutaneous aging and/or against the effects of stress inducing the variations observed during the process of cutaneous aging, and/or to prevent and/or fight against the diminishing of epidermal homeostasis, and/or to encourage epithelialization, and/or to improve cell proliferation and differentiation, notably at the epidermal level, and/or to prevent or fight against a diminishing of cutaneous vascularization, and/or to improve skin vascularization, and/or to improve vascular hyperpermeability, and/or to improve angiogenesis during scarring of the skin, and/or to prevent and/or fight against a diminishing of skin innervation, and/or to improve skin innervation, or to generate a sensation of well-being and/or to improve the color of the skin and/or to modify skin pigmentation, notably when the pigmentation presents localized flaws, such as pigmentary spots.
Embodiment 4. Use according to one of precedent embodiments wherein said substance stimulates the expression of MC-1R, and optionally maintains or stimulates POMC expression.
Embodiment 5. Use according to embodiment 4 to increase skin pigmentation, for tanning effect, to prevent and/or fight against depigmentation spots related or unrelated to aging.
Embodiment 6. Use according to embodiments 4 or 5, wherein the substance is chosen from among:
   - 1 methyl beta carbolin-3-carboxylic acid
   - Yeast fermented by lactic bacteria,
   - Lemon extract fermented by bifidobacterium,
   - Papaya extract fermented by lactic bacteria,
   - Lupin extract fermented by lactic bacteria,
   - Soy extract fermented by lactic bacteria,
   - Date extract fermented by lactic bacteria,
   - Extract of soy flour,
   - Extract of salsaparilla (Smilax ornata),
   - Extract of Cecropia obtusa,
   - Evening primrose extract *(Oenothera biennis),*
   - Achillea millefolium (common yarrow) extract,
   - Oenothera biennis (evening primrose) extract,
   - carrot extract,
   - male fern extract,
   - Prunus cerasus (sour cherry) extract, and
   - any mixture thereof.
Embodiment 7. Use according to any one of embodiments 1 to 3 wherein said substance decreases the expression of MC-1R, and optionally maintains or decreases POMC expression.
Embodiment 8. Use according to embodiment 7 to decrease skin pigmentation, for whitening or lightening effect, to increase brightness of tint, to prevent and/or fight against pigmentation spots related or unrelated to aging.
Embodiment 9. Use according to embodiments 7 or 8, wherein the substance is chosen from among:
   - An aqueous or hydroalcoolic extract of Juniperus communis (juniper berry),
   - An extract of Galeapsis ochroleuca
   - And any mixture thereof.
Embodiment 10. Use, according to any one of the preceding embodiments, characterized in that the said substance is used in combination with another active component chosen from among the group consisting of:
   - an active substance stimulating the proliferation and/or differentiation of keratinocytes, intended to have an anti-aging effect;
   - an active substance imitating the effect of beta endorphin in order to improve the skin's barrier function;
   - an active substance stimulating the trophicity of cutaneous nerves;
   - an active substance inducing a sensation of well-being;
   - an active substance permitting a change in the color of the skin,
   - an active substance stimulating the activity and/or proliferation of fibroblasts ,
   - an active substance protecting Fibroblast Growth Factor against degradation, notably FGF2,
   - an active substance stimulating Hyaluronase synthase, notably Hyaluronase synthase 2,
   - an active substance stimulating activity and/or synthesis of Lysyl Oxidase like LOXL,
   - an active with anti-inflammatory properties, notably inhibiting enzymatic activity of phospholipase A2,
   - an active substance with draining properties, and
   - any combination thereof.
Embodiment 11. Use of a substance chosen among :
   - acid 1 methyl beta carbolin 3 carboxylic;
   - (red colored) salsaparilla (Smilax ornata) extract,
   - Achillea millefolium (common yarrow) extract,
   - Cecropia obtuse extract,
   - Oenothera biennis (evening primrose) extract,
   - carrot extract,
   - male fern extract,
   - Prunus cerasus (sour cherry) extract,
   - Yeast fermented by lactic bacteria,
   - Lemon extract fermented by bifidobacterium,
   - Papaya extract fermented by lactic bacteria,
   - Lupin extract fermented by lactic bacteria,
   - Soy extract fermented by lactic bacteria,
   - Date extract fermented by lactic bacteria,
   - Extract of soy flour, and
   - any mixture thereof,
   as an active ingredient in a cosmetic composition or for the preparation of a neutraceutical compousition or for the preparation of a pharmaceutical notably dermatological composition intended to increase skin pigmentation, to exert a tanning effect, to prevent and/or fight against depigmentation spots related or unrelated to aging.
Embodiment 12. Use of a substance chosen among Galeopsis ochroleuca (wild hemp) or aqueous or hydroalcoolic extract of Juniperus communis (juniper berry) as an active ingredient in a cosmetic composition or for the preparation of a neutraceutical composition or for the preparation of a pharmaceutical notably dermatological composition, intended to decrease skin pigmentation, for whitening or lightening effect, to increase brightness of tint, to prevent and/or fight against pigmentation spots related or unrelated to aging.
Embodiment 13. A cosmetic or dermo-cosmetic or neutraceutic composition to prevent and/or fight against cutaneous aging and/or against the effects of stress inducing the variations observed during the process of cutaneous aging, or prevent and/or fight against the diminishing of epidermal homeostasis, or to encourage epithelialization, or to improve cellular proliferation and differentiation, notably at the epidermal level, or to prevent or fight against the diminishing of cutaneous vascularization, or to improve skin vascularization, or to improve vascular hyperpermeability, or to improve angiogenesis during scarring of the skin, or to prevent and/or fight against a diminishing of skin innervation, or to improve skin innervation, or to generate a sensation of well-being, or to improve the color of the skin and/or modify skin pigmentation, notably when the pigmentation presents localized flaws, such as pigmentary spots, the said composition including as an active ingredient an active substance such as defined in embodiments 6, 9, 11 or 12, possibly in combination with a substance such as described in embodiment 10.
Embodiment 14. A pharmaceutical composition, preferably intended to prevent and/or fight against cutaneous aging and/or against the effects of stress inducing variations observed during the process of cutaneous aging, or prevent and/or fight against the diminishing of epidermal homeostasis, or to encourage epithelialization, or to improve cellular proliferation and differentiation, notably at the epidermal level, or to prevent or fight against a diminishing of cutaneous vascularization, or to improve skin vascularization, or to improve vascular hyperpermeability, or to improve angiogenesis during skin scarring, or to prevent and/or fight against a diminishing of skin innervation, or to improve skin innervation, or to generate a sensation of well-being, or to improve the color of the skin and/or modify skin pigmentation, notably when the pigmentation presents localized flaws, such as pigmentary spots, the said composition including as an active ingredient an active substance such as defined in any of embodiments 6, 9, 11 or 12, possibly in combination with a substance as described in embodiment 10.
Embodiment 15. A method of cosmetic care including applying a substance as defined in embodiments 6, 9, 11 or 12 on the skin for improved esthetic effects, possibly in combination with a substance as described in embodiment 10.
Embodiment 16. A nucleotide sequence with one of the following sequences:
   GGGCTCTGAGAACGACTTTT (MC-1R Sense - SEQ ID N°7);
   CCGGGCTCCTGTCTGGTTGG (MC-1R Antisense - SEQ ID N°8);
   TCACGTCGCTGTTCCCGCTGAT (MC-2R Sense - SEQ ID N°9);
   AAGAGAGACATGTAGCAGGCGCAGTA (MC-2R Antisense - SEQ ID N°10);
   CTCAGCCAGGACTGGTTTCTGTAAGA (µ opioid receptor sense - SEQ ID N°11);
   TCGGACAGGTTGCCATCTAAGTG (µ opioid receptor Antisense - SEQ ID N°12);
   CGCCCAGTGAAGGTGTACCC (POMC Sense - SEQ ID N°13); and
   GGCGTCTGGCTCTTCTCGGAGGTC (POMC Antisense - SEQ ID N°14).

## Claims

1. Use of a common yarrow *(Achillea millefolium)* extract to stimulate the expression of receptor of a neuromediator coded by the POMC gene chosen from among MC-1R, MC-2R, and µ opioid R, in at least one type of skin cells expressing at least one of these receptors, wherein skin cells are chosen from among keratinocytes and/or nervous cells in cutaneous nervous network, as an active substance in a cosmetic or neutraceutic composition, or for the manufacture of a pharmaceutical, preferably dermatological composition.

2. Use of a common yarrow *(Achillea millefolium)* extract according to claim 1 in order to encourage at least one interaction of one chosen neuromediator among alpha-MSH, ACTH, and beta-endorphin, with their respective receptors.

3. Use of a common yarrow *(Achillea millefolium)* extract according to claim anyone of claims 1 to 2, to prevent and/or fight against the diminishing of epidermal thickness, preferably to prevent and/or fight against the diminishing of epidermal thickness during chronobiological or photo-induced cutaneous aging.

4. Use of a common yarrow *(Achillea millefolium)* extract according to anyone of claims 1 to 3 wherein the common yarrow *(Achillea millefolium)* extract increases or maintains the expression of the POMC gene to increase the effects of alpha-MSH, ACTH, and beta-endorphin.

5. Use of a common yarrow *(Achillea millefolium)* extract according to anyone of claims 1 to 4, to prevent and/or fight against cutaneous aging or the effects of stress inducing variations such as those observed to diminish during cutaneous aging.

6. Use of a common yarrow *(Achillea millefolium)* extract according to anyone of claims 1 to 5, to palliate a decrease in the interaction of alpha-MSH, and/or ACTH, and/or beta-endorphin with their respective receptors, linked to cellular aging and affecting keratinocytes and/or nervous cells, including cutaneous network.

7. Use of a common yarrow (Achillea millefolium) extract according to anyone of claims 1 to 6, wherein the common yarrow (Achillea millefolium) extract increases the synthesis of NGF and thus increases the growth of peripheral nerves at the cutaneous level; and preferably wherein the common yarrow (Achillea millefolium) extract also permits the increase of cutaneous innervation, observed to diminish during aging or under the effect of stress inducing variations such as those during the process of cutaneous aging.

8. Use of a common yarrow (Achillea millefolium) extract according to anyone of claims 1 to 7, wherein the common yarrow (Achillea millefolium) extract increases the synthesis of VEGF and thus increases the vascularization at the cutaneous level.

9. Use of a common yarrow (Achillea millefolium) extract according to claim 8, wherein the common yarrow (Achillea millefolium) extract permits better cutaneous irrigation, observed to diminish during aging or under the effect of stress inducing variations such as those during the process of cutaneous aging.

10. Use a common yarrow (Achillea millefolium) extract according to claim 1, wherein the composition is intended for cosmetic care or prophylactic or therapeutic treatment in order to improve hypermeability during skin scarring, improve cell proliferation and differentiation at the epidermal level, thus improving epithelialization of the skin and thus encouraging maintenance of skin innervation.

11. Use of a common yarrow *(Achillea millefolium)* extract to stimulate the expression of receptor of a neuromediator coded by the POMC gene chosen from among MC-1R and/or µ opioid R, in at least one type of skin cells expressing at least one of these receptors, wherein skin cells are melanocytes, as an active substance in a cosmetic or neutraceutic composition, or for the manufacture of a pharmaceutical, preferably dermatological composition, wherein the composition is to increase skin pigmentation, for tanning effect, to prevent and/or fight against depigmentation spots related or unrelated to aging.

12. Use of a common yarrow *(Achillea millefolium)* extract according to anyone of claims 1 to 13 wherein the common yarrow *(Achillea millefolium)* extract is obtained by macerating the common yarrow *(Achillea millefolium)* plant at 1-10% (p/p), in a solvent or a mixture of solvents.

13. Use of a common yarrow *(Achillea millefolium)* extract according to claim 14 wherein the solvent is chosen form among: water; a mixture of water and alcohol, glycol, or polyol 100/0 to 0/100; and preferably wherein the solvent is water.

14. Use of a common yarrow *(Achillea millefolium)* extract according to anyone of claims 1 to 13, as an active substance in a cosmetic composition, wherein the common yarrow *(Achillea millefolium)* extract is used in the cosmetic composition in an amount comprised between 0,01% and 5% (v/v).

15. Use of a common yarrow *(Achillea millefolium)* extract according to claim 1 for the manufacture of a pharmaceutical, preferably dermatological composition intended for prophylactic or therapeutic treatment in order to improve innervation, to prevent and/or fight against pathologies due to, associated with and/or inducing lost of number and/or activity of nervous cells, notably in case of scaring and/or wound healing, stretchmarks, burns, psoriasis or dermatitis.


SEQUENCE LISTING

<110>  BASF BEAUTY CARE SOLUTIONS FRANCE SAS

<120>  MC-1R, MC-2R, and μ opioïd receptors modulation

<130>  H197810/68 PCT2

<150>  FR 07 55529
<151>  2007-06-06

<160>  14

<170>  PatentIn version 3.3

<210>  1
<211>  2360
<212>  DNA
<213>  Homo sapiens

<400>  1
gagagggcag gtcccgggga agctccggac tcctagaggg gcggccaggt gggggccctg      60

gtgaccagga cagactgtgg tgttttttaa cgtaaaggag atccgcggtg tgagggaccc     120

cctgggtcct gcacgccgcc tggtggcagg ccgggccatg gtgggtgctc acgcccccgg     180

catgtggccg ccctcagtgg gaggggctct gagaacgact ttttaaaacg cagagaaaag     240

ctccattctt cccaggacct cagcgcagcc ctggcccagg aaggcaggag acagaggcca     300

ggacggtcca gaggtgtcga aatgtcctgg ggacctgagc agcagccacc agggaagagg     360

cagggaggga gctgaggacc aggcttggtt gtgagaatcc ctgagcccag gcggtagatg     420

ccaggaggtg tctggactgg ctgggccatg cctgggctga cctgtccagc cagggagagg     480

gtgtgagggc agatctgggg gtgcccagat ggaaggaggc aggcatgggg gacacccaag     540

gccccctggc agcaccatga actaagcagg acacctggag gggaagaact gtggggacct     600

ggaggcctcc aacgactcct tcctgcttcc tggacaggac tatggctgtg cagggatccc     660

agagaagact tctgggctcc ctcaactcca cccccacagc catcccccag ctggggctgg     720

ctgccaacca gacaggagcc cggtgcctgg aggtgtccat ctctgacggg ctcttcctca     780

gcctggggct ggtgagcttg gtggagaacg cgctggtggt ggccaccatc gccaagaacc     840

ggaacctgca ctcacccatg tactgcttca tctgctgcct ggccttgtcg gacctgctgg     900

tgagcgggag caacgtgctg gagacggccg tcatcctcct gctggaggcc ggtgcactgg     960

tggcccgggc tgcggtgctg cagcagctgg acaatgtcat tgacgtgatc acctgcagct    1020

ccatgctgtc cagcctctgc ttcctgggcg ccatcgccgt ggaccgctac atctccatct    1080

tctacgcact gcgctaccac agcatcgtga ccctgccgcg ggcgcggcga gccgttgcgg    1140

ccatctgggt ggccagtgtc gtcttcagca cgctcttcat cgcctactac gaccacgtgg    1200

ccgtcctgct gtgcctcgtg gtcttcttcc tggctatgct ggtgctcatg gccgtgctgt 1260

acgtccacat gctggcccgg gcctgccagc acgcccaggg catcgcccgg ctccacaaga 1320

ggcagcgccc ggtccaccag ggctttggcc ttaaaggcgc tgtcaccctc accatcctgc 1380

tgggcatttt cttcctctgc tggggcccct tcttcctgca tctcacactc atcgtcctct 1440

gccccgagca ccccacgtgc ggctgcatct tcaagaactt caacctcttt ctcgccctca 1500

tcatctgcaa tgccatcatc gaccccctca tctacgcctt ccacagccag gagctccgca 1560

ggacgctcaa ggaggtgctg acatgctcct ggtgagcgcg gtgcacgcgg ctttaagtgt 1620

gctgggcaga gggaggtggt gatattgtgt ggtctggttc ctgtgtgacc ctgggcagtt 1680

ccttacctcc ctggtccccg tttgtcaaag aggatggact aaatgatctc tgaaagtgtt 1740

gaagcgcgga cccttctggg tccagggagg ggtccctgca aaactccagg caggacttct 1800

caccagcagt cgtggggaac ggaggaggac atggggaggt tgtggggcct caggctccgg 1860

gcaccagggg ccaacctcag gctcctaaag agacattttc cgcccactcc tgggacactc 1920

cgtctgctcc aatgactgag cagcatccac cccaccccat ctttgctgcc agctctcagg 1980

accgtgccct cgtcagctgg gatgtgaagt ctctgggtgg aagtgtgtgc caagagctac 2040

tcccacagca gccccaggag aaggggcttt gtgaccagaa agcttcatcc acagccttgc 2100

agcggctcct gcaaaaggag gtgaaatccc tgcctcaggc caagggacca ggtttgcagg 2160

agccccccta gtggtatggg gctgagccct cctgagggcc ggttctaagg ctcagactgg 2220

gcactggggc ctcagcctgc tttcctgcag cagtcgccca agcagacagc cctggcaaat 2280

gcctgactca gtgaccagtg cctgtgagca tggggccagg aaagtctggt aataaatgtg 2340

actcagcatc acccacctta 2360

<210> 2
<211> 894
<212> DNA
<213> Homo sapiens

<400> 2
atgaagcaca ttatcaactc gtatgaaaac atcaacaaca cagcaagaaa taattccgac 60

tgtcctcgtg tggttttgcc ggaggagata tttttcacaa tttccattgt tggagttttg 120

gagaatctga tcgtcctgct ggctgtgttc aagaataaga atctccaggc acccatgtac 180

tttttcatct gtagcttggc catatctgat atgctgggca gcctatataa gatcttggaa 240

aatatcctga tcatattgag aaacatgggc tatctcaagc cacgtggcag ttttgaaacc 300

acagccgatg acatcatcga ctccctgttt gtcctctccc tgcttggctc catcttcagc 360

ctgtctgtga ttgctgcgga ccgctacatc accatcttcc acgcactgcg gtaccacagc 420

atcgtgacca tgcgccgcac tgtggtggtg cttacggtca tctggacgtt ctgcacgggg 480

actggcatca ccatggtgat cttctcccat catgtgccca cagtgatcac cttcacgtcg 540

ctgttcccgc tgatgctggt cttcatcctg tgcctctatg tgcacatgtt cctgctggct 600

cgatcccaca ccaggaagat ctccaccctc cccagagcca acatgaaagg ggccatcaca 660

ctgaccatcc tgctcggggt cttcatcttc tgctgggccc cctttgtgct tcatgtcctc 720

ttgatgacat tctgcccaag taacccctac tgcgcctgct acatgtctct cttccaggtg 780

aacggcatgt tgatcatgtg caatgccgtc attgacccct tcatatatgc cttccggagc 840

ccagagctca gggacgcatt caaaaagatg atcttctgca gcaggtactg gtag 894

<210> 3
<211> 1891
<212> DNA
<213> homo sapiens

<400> 3
gatgagcctc tgtgaactac taaggtggga gggggctata cgcagaggag aatgtcagat 60

gctcagctcg gtcccctccg cctgacgctc ctctctgtct cagccaggac tggtttctgt 120

aagaaacagc aggagctgtg gcagcggcga aaggaagcgg ctgaggcgct tggaacccga 180

aaagtctcgg tgctcctggc tacctcgcac agcggtgccc gcccggccgt cagtaccatg 240

gacagcagcg ctgcccccac gaacgccagc aattgcactg atgccttggc gtactcaagt 300

tgctccccag cacccagccc cggttcctgg gtcaacttgt cccacttaga tggcaacctg 360

tccgacccat gcggtccgaa ccgcaccgac ctgggcggga gagacagcct gtgccctccg 420

accggcagtc cctccatgat cacggccatc acgatcatgg ccctctactc catcgtgtgc 480

gtggtggggc tcttcggaaa cttcctggtc atgtatgtga ttgtcagata caccaagatg 540

aagactgcca ccaacatcta cattttcaac cttgctctgg cagatgcctt agccaccagt 600

accctgccct tccagagtgt gaattaccta atgggaacat ggccatttgg aaccatcctt 660

tgcaagatag tgatctccat agattactat aacatgttca ccagcatatt caccctctgc 720

accatgagtg ttgatcgata cattgcagtc tgccaccctg tcaaggcctt agatttccgt 780

actccccgaa atgccaaaat tatcaatgtc tgcaactgga tcctctcttc agccattggt 840

cttcctgtaa tgttcatggc tacaacaaaa tacaggcaag gttccataga ttgtacacta 900

acattctctc atccaacctg gtactgggaa aacctgctga agatctgtgt tttcatcttc 960

gccttcatta tgccagtgct catcattacc gtgtgctatg gactgatgat cttgcgcctc 1020

aagagtgtcc gcatgctctc tggctccaaa gaaaaggaca ggaatcttcg aaggatcacc 1080

aggatggtgc tggtggtggt ggctgtgttc atcgtctgct ggactcccat tcacatttac 1140

gtcatcatta aagccttggt tacaatccca gaaactacgt tccagactgt ttcttggcac 1200

ttctgcattg ctctaggtta cacaaacagc tgcctcaacc cagtccttta tgcatttctg 1260

gatgaaaact tcaaacgatg cttcagagag ttctgtatcc caacctcttc caacattgag 1320

caacaaaact ccactcgaat tcgtcagaac actagagacc acccctccac ggccaataca 1380

gtggatagaa ctaatcatca gctagaaaat ctggaagcag aaactgctcc gttgccctaa 1440

cagggtctca tgccattccg accttcacca agcttagaag ccaccatgta tgtggaagca 1500

ggttgcttca agaatgtgta ggaggctcta attctctagg aaagtgcctg cttttaggtc 1560

atccaacctc tttcctctct ggccactctg ctctgcacat tagagggaca gccaaaagta 1620

agtggagcat ttggaaggaa aggaatatac cacaccgagg agtccagttt gtgcaagaca 1680

cccagtggaa ccaaaaccca tcgtggtatg tgaattgaag tcatcataaa aggtgaccct 1740

tctgtctgta agattttatt ttcaagcaaa tatttatgac ctcaacaaag aagaaccatc 1800

ttttgttaag ttcaccgtag taacacataa agtaaatgct acctctgatc aaagcacctt 1860

gaatggaagg tccgagtctt tttagtgttt t 1891

<210> 4
<211> 1071
<212> DNA
<213> homo sapiens

<400> 4
agcggcggcg aaggagggga agaagagccg cgaccgagag aggccgccga gcgtccccgc 60

cctcagagag cagcctcccg agacagagcc tcagcctgcc tggaagatgc cgagatcgtg 120

ctgcagccgc tcgggggccc tgttgctggc cttgctgctt caggcctcca tggaagtgcg 180

tggctggtgc ctggagagca gccagtgtca ggacctcacc acggaaagca acctgctgga 240

gtgcatccgg gcctgcaagc ccgacctctc ggccgagact cccatgttcc cgggaaatgg 300

cgacgagcag cctctgaccg agaacccccg gaagtacgtc atgggccact tccgctggga 360

ccgattcggc cgccgcaaca gcagcagcag cggcagcagc ggcgcagggc agaagcgcga 420

ggacgtctca gcgggcgaag actgcggccc gctgcctgag ggcggccccg agccccgcag 480

cgatggtgcc aagccgggcc cgcgcgaggg caagcgctcc tactccatgg agcacttccg 540

ctggggcaag ccggtgggca agaagcggcg cccagtgaag gtgtacccta acggcgccga 600

ggacgagtcg gccgaggcct tccccctgga gttcaagagg gagctgactg gccagcgact 660

ccgggaggga gatggccccg acggccctgc cgatgacggc gcaggggccc aggccgacct 720

ggagcacagc ctgctggtgg cggccgagaa gaaggacgag ggcccctaca ggatggagca 780

cttccgctgg ggcagcccgc ccaaggacaa gcgctacggc ggtttcatga cctccgagaa 840

gagccagacg cccctggtga cgctgttcaa aaacgccatc atcaagaacg cctacaagaa    900

gggcgagtga gggcacagcg ggccccaggg ctaccctccc ccaggaggtc gaccccaaag    960

ccccttgctc tcccctgccc tgctgccgcc tcccagcctg gggggtcgtg gcagataatc   1020

agcctcttaa agctgcctgt agttaggaaa taaaaccttt caaatttcac a            1071

<210>  5
<211>  20
<212>  DNA
<213>  artificial sequence

<220>
<223>  sonde Actine Sens

<400>  5
gtggggcgcc ccaggcacca                                                 20

<210>  6
<211>  24
<212>  DNA
<213>  artificial sequence

<220>
<223>  sonde Actine Antisens

<400>  6
ctccttaatg tcacgcacga tttc                                            24

<210>  7
<211>  20
<212>  DNA
<213>  artificial sequence

<220>
<223>  Sonde MCR sens

<400>  7
gggctctgag aacgactttt                                                 20

<210>  8
<211>  20
<212>  DNA
<213>  artificial sequence

<220>
<223>  sonde MCR1 Antisens

<400>  8
ccgggctcct gtctggttgg                                                 20

<210>  9
<211>  22
<212>  DNA
<213>  artificial sequence

<220>
<223>  sonde MCR2 Sens

<400>  9
tcacgtcgct gttcccgctg at                                              22

<210>  10
<211>  26
<212>  DNA
<213>  artificial sequence

<220>
<223>  sonde MCR2 Antisens

<400>  10
aagagagaca tgtagcaggc gcagta                                          26

<210>  11
<211>  26
<212>  DNA
<213>  artificial sequence

<220>
<223>  sonde µ opioide Sens

<400>  11
ctcagccagg actggtttct gtaaga                                          26

<210>  12
<211>  23
<212>  DNA
<213>  artificial sequence

<220>
<223>  sonde µ opioide Antisens

<400>  12
tcggacaggt tgccatctaa gtg                                             23

<210>  13
<211>  20
<212>  DNA
<213>  artificial sequence

<220>
<223>  sonde POMC Sens

<400>  13
cgcccagtga aggtgtaccc                                                 20

<210>  14
<211>  24
<212>  DNA
<213>  artificial sequence

<220>
<223>  sonde POMC Antisens

<400>  14
ggcgtctggc tcttctcgga ggtc                                              24